# EUROPEAN PATENT APPLICATION

(11) **EP 3 760 722 A1**
(43) Date of publication of application: **06.01.2021**
(21) Application number: 19760224.6
(22) Date of filing: 01.03.2019
(51) Int. Cl.: C12N 15/13, A61K 31/7088, A61K 35/76, A61K 39/395, A61K 48/00, A61P 1/16, A61P 1/18, A61P 9/00, A61P 11/00, A61P 13/12, C07K 16/28, C12N 15/63

(54) **ANTI-INTEGRIN ALPHA11 MONOCLONAL ANTIBODY AND USE THEREOF**

(30) Priority: 01.03.2018 JP 2018036933
(71) Applicant: Hiroshima University, Higashi-Hiroshima-shi Hiroshima 739-8511 (JP)
(72) Inventor: YOKOSAKI, Yasuyuki, Hiroshima-shi, Hiroshima 734-8551 (JP); NISHIMICHI, Norihisa, Hiroshima-shi, Hiroshima 734-8551 (JP)
(74) Representative: Schön, Christoph
(86) International application number: PCT/JP2019/008202
(87) International publication number: WO 2019/168176

(57) **Abstract**

The present invention provides a novel anti-integrin α11 monoclonal antibody that may treat a fibrotic disease. An aspect of the present invention is an anti-integrin α11 monoclonal antibody which inhibits binding between an integrin α11 and collagen.

SEQUENCE LISTING

<110>  Hiroshima University

<120>  ANTI-INTEGRIN ALPHA 11 MONOCLONAL ANTIBODY, AND USE THEREOF

<130>  HU19008

<150>  JP 2018-036933
<151>  2018-03-01

<160>  64

<170>  PatentIn version 3.5

<210>  1
<211>  5
<212>  PRT
<213>  Gallus gallus

<400>  1

Ser Tyr Gly Met Gln
1               5

<210>  2
<211>  17
<212>  PRT
<213>  Gallus gallus

<400>  2

Gly Ile Asp Asp Asp Gly Ser Phe Thr Leu Tyr Gly Ala Ala Val Asp
1               5                   10                  15

Gly

<210>  3
<211>  16
<212>  PRT
<213>  Gallus gallus

<400>  3

Gly Gly Tyr Gly Tyr Cys Trp Tyr Gly Asp Ala Ala Cys Ile Asp Ala
1               5                   10                  15

<210>  4
<211>  9
<212>  PRT
<213>  Gallus gallus

<400>  4

Ser Gly Gly Ser Asp Ser Trp Tyr Gly
1               5

<210>  5
<211>  7
<212>  PRT
<213>  Gallus gallus

<400>  5

Ser Asn Asn Gln Arg Pro Ser
1               5


<210>  6
<211>  10
<212>  PRT
<213>  Gallus gallus

<400>  6

Gly Ser Arg Asp Ser Ser Tyr Asp Gly Ile
1               5                   10


<210>  7
<211>  125
<212>  PRT
<213>  Gallus gallus

<400>  7

Ala Val Thr Leu Asp Glu Ser Gly Gly Gly Leu Gln Thr Pro Gly Gly
1               5                   10                  15


Ala Leu Ser Leu Val Cys Lys Gly Ser Gly Phe Thr Phe Ser Ser Tyr
            20                  25                  30


Gly Met Gln Trp Val Arg Gln Ala Pro Gly Arg Gly Leu Glu Trp Val
        35                  40                  45


Ala Gly Ile Asp Asp Asp Gly Ser Phe Thr Leu Tyr Gly Ala Ala Val
    50                  55                  60


Asp Gly Arg Ala Thr Ile Ser Arg Asp Asn Gly Gln Thr Thr Val Arg
65                  70                  75                  80


Leu Gln Leu Ser Asp Leu Arg Ala Glu Asp Thr Ala Thr Tyr Phe Cys
                85                  90                  95


Ala Lys Gly Gly Tyr Gly Tyr Cys Trp Tyr Gly Asp Ala Ala Cys Ile
            100                 105                 110


Asp Ala Trp Gly His Gly Thr Glu Val Ile Val Ser Ser
        115                 120                 125


<210>  8
<211>  105

<212>  PRT
<213>  Gallus gallus

<400>  8

Ala Val Thr Gln Pro Ser Ser Val Ser Ala Asn Pro Gly Glu Thr Val
1               5                   10                  15

Lys Ile Thr Cys Ser Gly Gly Ser Asp Ser Trp Tyr Gly Trp Tyr Gln
            20                  25                  30

Gln Lys Ser Pro Gly Ser Ala Pro Val Thr Val Ile Tyr Ser Asn Asn
        35                  40                  45

Gln Arg Pro Ser Asn Ile Pro Ser Arg Phe Ser Gly Ser Thr Ser Gly
    50                  55                  60

Ser Thr Ser Thr Leu Thr Ile Thr Gly Val Gln Ala Asp Asp Glu Ala
65                  70                  75                  80

Val Tyr Phe Cys Gly Ser Arg Asp Ser Ser Tyr Asp Gly Ile Phe Gly
                85                  90                  95

Ala Gly Thr Thr Leu Thr Val Leu Lys
            100                 105

<210>  9
<211>  31
<212>  DNA
<213>  Artificial sequence

<220>
<223>  primer

<400>  9
cctggtgcag gcacagactt acatggacat c                                31

<210>  10
<211>  25
<212>  DNA
<213>  Artificial sequence

<220>
<223>  primer

<400>  10
gcaccggtca tctccagccc aaaag                                       25

<210>  11
<211>  31
<212>  DNA
<213>  Artificial sequence

<220>

<223>  primer

<400>  11
atggatccgc catggcctgg gctcctctcc t                                    31


<210>  12
<211>  20
<212>  DNA
<213>  Artificial sequence

<220>
<223>  primer

<400>  12
tgcctgcacc agggaacctg                                                 20


<210>  13
<211>  20
<212>  DNA
<213>  Artificial sequence

<220>
<223>  primer

<400>  13
ctgatggcgg ccgtgacgtt                                                 20


<210>  14
<211>  27
<212>  DNA
<213>  Artificial sequence

<220>
<223>  primer

<400>  14
tccacctgtc gacacggtga cttcggt                                         27


<210>  15
<211>  23
<212>  DNA
<213>  Artificial sequence

<220>
<223>  primer

<400>  15
tctgacgtcg cgctgactca gcc                                             23


<210>  16
<211>  28
<212>  DNA
<213>  Artificial sequence

<220>
<223>  primer

<400>  16

attagcgcgc ttaaggacgg tcagggtt                                          28


<210>  17
<211>  69
<212>  DNA
<213>  Artificial sequence

<220>
<223>  primer

<400>  17
ccgtgtcgac aggtggaggc ggctctggtg gcggtggcag tggcggcgga ggttctgacg      60

tcgcgctga                                                              69


<210>  18
<211>  69
<212>  DNA
<213>  Artificial sequence

<220>
<223>  primer

<400>  18
tcagcgcgac gtcagaacct ccgccgccac tgccaccgcc accagagccg cctccacctg      60

tcgacacgg                                                              69


<210>  19
<211>  19
<212>  DNA
<213>  Artificial sequence

<220>
<223>  primer

<400>  19
tatctgatgg cggccgtga                                                   19


<210>  20
<211>  22
<212>  DNA
<213>  Artificial sequence

<220>
<223>  primer

<400>  20
gactatatta gcgcgcttaa gg                                               22


<210>  21
<211>  19
<212>  DNA
<213>  Artificial sequence

<220>
<223>  primer

<400>  21
ggaaacagct atgaccatg                                                    19


<210>  22
<211>  17
<212>  DNA
<213>  Artificial sequence

<220>
<223>  primer

<400>  22
aagaagcaca cgactga                                                      17


<210>  23
<211>  23
<212>  DNA
<213>  Artificial sequence

<220>
<223>  primer

<400>  23
attgtcctgg atggctccaa cag                                               23


<210>  24
<211>  21
<212>  DNA
<213>  Artificial sequence

<220>
<223>  primer

<400>  24
aatgatgtcc atgtaggtct g                                                 21


<210>  25
<211>  24
<212>  DNA
<213>  Artificial sequence

<220>
<223>  primer

<400>  25
gcagctgcca gccacattga gcag                                              24


<210>  26
<211>  22
<212>  DNA
<213>  Artificial sequence

<220>
<223>  primer

<400>  26
caccacatct tttacagacc tg                                                22

<210>  27
<211>  23
<212>  DNA
<213>  Artificial sequence

<220>
<223>  primer

<400>  27
tacggcattg aatttgcacg ctc                                               23


<210>  28
<211>  23
<212>  DNA
<213>  Artificial sequence

<220>
<223>  primer

<400>  28
tgccgtccgg gtctctgttc ctc                                               23


<210>  29
<211>  23
<212>  DNA
<213>  Artificial sequence

<220>
<223>  primer

<400>  29
gatagcgaaa gagacaacgt aac                                               23


<210>  30
<211>  23
<212>  DNA
<213>  Artificial sequence

<220>
<223>  primer

<400>  30
ctggatcacc ttctccaggt ctg                                               23


<210>  31
<211>  24
<212>  DNA
<213>  Artificial sequence

<220>
<223>  primer

<400>  31
gagagaatct tcagcctgga aggc                                              24


<210>  32
<211>  22

<212>  DNA
<213>  Artificial sequence

<220>
<223>  primer

<400>  32
ccccagggca tcgacaatgt cc                                                22


<210>  33
<211>  21
<212>  DNA
<213>  Artificial sequence

<220>
<223>  primer

<400>  33
atctcctttg ggctggagat g                                                 21


<210>  34
<211>  22
<212>  DNA
<213>  Artificial sequence

<220>
<223>  primer

<400>  34
ctcgttcttg ttggtgcctt cc                                                22


<210>  35
<211>  35
<212>  DNA
<213>  Artificial sequence

<220>
<223>  primer

<400>  35
ccccacaggg ctgatggcgg ccgtgacgtt ggacg                                  35


<210>  36
<211>  21
<212>  DNA
<213>  Artificial sequence

<220>
<223>  primer

<400>  36
ggaggagacg atgacttcgg t                                                 21


<210>  37
<211>  35
<212>  DNA
<213>  Artificial sequence

<220>
<223>  primer

<400>  37
aggttccctg gtgcaggcag cagtgactca gccgc                                35


<210>  38
<211>  32
<212>  DNA
<213>  Artificial sequence

<220>
<223>  primer

<400>  38
atctagatta acactcattc ctgttgaagc tc                                   32


<210>  39
<211>  29
<212>  DNA
<213>  Artificial sequence

<220>
<223>  primer

<400>  39
ttaagcttac catgagccca ctcgtctcc                                       29


<210>  40
<211>  20
<212>  DNA
<213>  Artificial sequence

<220>
<223>  primer

<400>  40
cgccatcagc cctgtgggga                                                 20


<210>  41
<211>  29
<212>  DNA
<213>  Artificial sequence

<220>
<223>  primer

<400>  41
gcaagcttgc catggcctgg gctcctctc                                       29


<210>  42
<211>  20
<212>  DNA
<213>  Artificial sequence

<220>
<223>  primer

<400>  42
tgcctgcacc agggaacctg                                                   20


<210>  43
<211>  32
<212>  DNA
<213>  Artificial sequence

<220>
<223>  primer

<400>  43
gtcatcgtct cctccgccaa aacgacaccc cc                                     32


<210>  44
<211>  21
<212>  DNA
<213>  Artificial sequence

<220>
<223>  primer

<400>  44
tcttgtccac cttggtgctg c                                                 21


<210>  45
<211>  21
<212>  DNA
<213>  Artificial sequence

<220>
<223>  primer

<400>  45
gacaatggct ccgggctctg t                                                 21


<210>  46
<211>  20
<212>  DNA
<213>  Artificial sequence

<220>
<223>  primer

<400>  46
tgccgtgttc tatcggatac                                                   20


<210>  47
<211>  20
<212>  DNA
<213>  Artificial sequence

<220>
<223>  primer

<400>  47
tgtggtgttg aggaaagcag                                                   20

<210>  48
<211>  20
<212>  DNA
<213>  Artificial sequence

<220>
<223>  primer

<400>  48
tggccagaac ctggctatac                                                   20


<210>  49
<211>  20
<212>  DNA
<213>  Artificial sequence

<220>
<223>  primer

<400>  49
ttcaatgtcc cagccatgta                                                   20


<210>  50
<211>  20
<212>  DNA
<213>  Artificial sequence

<220>
<223>  primer

<400>  50
gaaggaatag ccacgctcag                                                   20


<210>  51
<211>  20
<212>  DNA
<213>  Artificial sequence

<220>
<223>  primer

<400>  51
gaggatgagg tggaacgtgt                                                   20


<210>  52
<211>  20
<212>  DNA
<213>  Artificial sequence

<220>
<223>  primer

<400>  52
tcttcagtcg ctccaggtct                                                   20


<210>  53
<211>  20

<212>  DNA
<213>  Artificial sequence

<220>
<223>  primer

<400>  53
aatggctctg ggctctgtaa                                                    20


<210>  54
<211>  20
<212>  DNA
<213>  Artificial sequence

<220>
<223>  primer

<400>  54
ctcttgctct gggcttcatc                                                    20


<210>  55
<211>  20
<212>  DNA
<213>  Artificial sequence

<220>
<223>  primer

<400>  55
tgtgtccgtc gtggatctga                                                    20


<210>  56
<211>  21
<212>  DNA
<213>  Artificial sequence

<220>
<223>  primer

<400>  56
ttgctgttga agtcgcagga g                                                  21


<210>  57
<211>  16
<212>  PRT
<213>  Artificial sequence

<220>
<223>  modified sequence

<400>  57

Gly Ser Ala Gly Trp Cys Trp Met Gly Asp Ala Ala Cys Ile Asp Ala
1               5                   10                  15


<210>  58
<211>  16
<212>  PRT

<213>  Artificial sequence

<220>
<223>  modified sequence

<400>  58

Gly His Thr Asp Trp Cys Trp Met Gly Asp Ala Ala Cys Ile Asp Ala
1               5                   10                  15


<210>  59
<211>  16
<212>  PRT
<213>  Artificial sequence

<220>
<223>  modified sequence

<400>  59

Gly Glu Phe Gly Trp Cys Trp Met Gly Asp Ala Ala Cys Ile Asp Ala
1               5                   10                  15


<210>  60
<211>  16
<212>  PRT
<213>  Artificial sequence

<220>
<223>  modified sequence

<400>  60

Gly Ser Ser Val Asp Cys Trp Met Gly Asp Ala Ala Cys Ile Asp Ala
1               5                   10                  15


<210>  61
<211>  16
<212>  PRT
<213>  Artificial sequence

<220>
<223>  modified sequence

<400>  61

Gly Ser Met Gly Trp Cys Trp Met Gly Asp Ala Ala Cys Ile Asp Ala
1               5                   10                  15


<210>  62
<211>  16
<212>  PRT
<213>  Artificial sequence

<220>
<223>  modified sequence

<400>  62

Gly Ser Glu Ala Trp Cys Trp Met Gly Asp Ala Ala Cys Ile Asp Ala
1               5                   10                  15

<210>  63
<211>  61
<212>  DNA
<213>  Artificial sequence

<220>
<221>  misc_feature
<222>  (19)..(20)
<223>  n is a, c, g, or t

<220>
<221>  misc_feature
<222>  (21)..(21)
<223>  s is c or g

<220>
<221>  misc_feature
<222>  (22)..(23)
<223>  n is a, c, g, or t

<220>
<221>  misc_feature
<222>  (24)..(24)
<223>  s is c or g

<220>
<221>  misc_feature
<222>  (25)..(26)
<223>  n is a, c, g, or t

<220>
<221>  misc_feature
<222>  (27)..(27)
<223>  s is c or g

<220>
<221>  misc_feature
<222>  (28)..(29)
<223>  n is a, c, g, or t

<220>
<221>  misc_feature
<222>  (30)..(30)
<223>  s is c or g

<220>
<221>  misc_feature
<222>  (34)..(35)
<223>  n is a, c, g, or t

<220>
<221>  misc_feature
<222>  (36)..(36)
<223>  s is c or g

<220>
<221>  misc_feature
<222>  (37)..(38)

<223>  n is a, c, g, or t

<220>
<221>  misc_feature
<222>  (39)..(39)
<223>  s is c or g

<400>  63
tacttctgcg ccaagggtnn snnsnnsnns tgtnnsnnsg gtgatgctgc ttgtattgac        60

g                                                                         61


<210>  64
<211>  27
<212>  DNA
<213>  Artificial sequence

<220>
<223>  primer

<400>  64
ggtggcggtg tcctcagccc tgaggtc                                             27

## Description

### Technical Field

The present invention relates to an anti-integrin α11 monoclonal antibody, a polynucleotide including a base sequence which encodes the antibody, and a vector including the polynucleotide. The present invention also relates to use of the antibody, the polynucleotide or the vector.

### Background Art

An integrin α 11 subunit (hereinafter sometimes referred to simply as "integrin α11" or "α11") is one of cell adhesion molecules that form a dimer with an integrin β1 subunit and have binding activity with collagen species.

The integrin α11 is known to be involved in tissue fibrosis (Non-patent Literature 1). Therefore, the integrin α11 is considered to be an effective treatment target of a disease associated with tissue fibrosis, e.g., a fibrotic disease, and attempts have been made to develop a medicine targeting the integrin α11 (Non-patent Literature 2). For example, it is reported in Non-patent Literature 2 that transition to cirrhosis could be prevented by inhibiting expression of an integrin α11 using siRNA.

### Citation List

### [Non-patent Literature]

[Non-patent Literature 1]
   Sergio Carracedo et. al., J Biol Chem., 285(14): 10434-10445, 2010.
[Non-patent Literature 2]
   Ruchi Bansal et. al., Exp Mol Med., 49(11): e396, 2017.

### Summary of Invention

### Technical Problem

Until now, no monoclonal antibody has been found which binds to an integrin α11 and is applicable to treatment of the fibrotic disease.

An object of an aspect of the present invention is to provide a novel anti-integrin α11 monoclonal antibody.

### Solution to Problem

As a result of diligent studies to attain the above object, the inventors of the present invention succeeded in obtaining a novel anti-integrin α11 monoclonal antibody and have completed the present invention by identifying an amino acid sequence of the anti-integrin α11 monoclonal antibody. That is, an embodiment of the present invention encompasses the following features:
<1> An anti-integrin α11 monoclonal antibody which inhibits binding between an integrin α11 and collagen.
<2> The anti-integrin α11 monoclonal antibody described in <1> which has at least one activity selected from the group consisting of cell adhesion inhibitory activity and cell detachment activity.
<3> The anti-integrin α11 monoclonal antibody described in <1> or <2> which specifically binds to an integrin α11.
<4> The anti-integrin α11 monoclonal antibody described in any one of <1> through <3> which specifically binds to integrins α11 derived from two or more different species of mammals.
<5> The anti-integrin α11 monoclonal antibody described in any one of <1> through <4>, including:
   (a) a heavy chain variable region CDR1 which is any of the following (a1) through (a3):
      (a1) a polypeptide having an amino acid sequence of SEQ ID NO: 1;
      (a2) a polypeptide having an amino acid sequence in which one or more amino acids are deleted, substituted, or added in the amino acid sequence of SEQ ID NO: 1, and having a function as the heavy chain variable region CDR1;
      (a3) a polypeptide having an amino acid sequence that has homology of 80% or higher with respect to the amino acid sequence of SEQ ID NO: 1, and having a function as the heavy chain variable region CDR1;
   (b) a heavy chain variable region CDR2 which is any of the following (b1) through (b3):
      (b1) a polypeptide having an amino acid sequence of SEQ ID NO: 2;
      (b2) a polypeptide having an amino acid sequence in which one or more amino acids are deleted, substituted, or added in the amino acid sequence of SEQ ID NO: 2, and having a function as the heavy chain variable region CDR2;
      (b3) a polypeptide having an amino acid sequence that has homology of 80% or higher with respect to the amino acid sequence of SEQ ID NO: 2, and having a function as the heavy chain variable region CDR2;
   (c) a heavy chain variable region CDR3 which is any of the following (c1) through (c3):
      (c1) a polypeptide having an amino acid sequence of SEQ ID NO: 3;
      (c2) a polypeptide having an amino acid sequence in which one or more amino acids are deleted, substituted, or added in the amino acid sequence of SEQ ID NO: 3, and having a function as the heavy chain variable region CDR3;
      (c3) a polypeptide having an amino acid sequence that has homology of 80% or higher with respect to the amino acid sequence of SEQ ID NO: 3, and having a function as the heavy chain variable region CDR3;
   (d) a light chain variable region CDR1 which is any of the following (d1) through (d3):
      (d1) a polypeptide having an amino acid sequence of SEQ ID NO: 4;
      (d2) a polypeptide having an amino acid sequence in which one or more amino acids are deleted, substituted, or added in the amino acid sequence of SEQ ID NO: 4, and having a function as the light chain variable region CDR1;
      (d3) a polypeptide having an amino acid sequence that has homology of 80% or higher with respect to the amino acid sequence of SEQ ID NO: 4, and having a function as the light chain variable region CDR1;
   (e) a light chain variable region CDR2 which is any of the following (e1) through (e3):
      (e1) a polypeptide having an amino acid sequence of SEQ ID NO: 5;
      (e2) a polypeptide having an amino acid sequence in which one or more amino acids are deleted, substituted, or added in the amino acid sequence of SEQ ID NO: 5, and having a function as the light chain variable region CDR2;
      (e3) a polypeptide having an amino acid sequence that has homology of 80% or higher with respect to the amino acid sequence of SEQ ID NO: 5, and having a function as the light chain variable region CDR2; and
   (f) a light chain variable region CDR3 which is any of the following (f1) through (f3):
      (f1) a polypeptide having an amino acid sequence of SEQ ID NO: 6;
      (f2) a polypeptide having an amino acid sequence in which one or more amino acids are deleted, substituted, or added in the amino acid sequence of SEQ ID NO: 6, and having a function as the light chain variable region CDR3;
      (f3) a polypeptide having an amino acid sequence that has homology of 80% or higher with respect to the amino acid sequence of SEQ ID NO: 6, and having a function as the light chain variable region CDR3.
<6> The anti-integrin α11 monoclonal antibody described in any one of <1> through <5>, including:
   (g) a heavy chain variable region which is any of the following (g1) through (g3):
      (g1) a polypeptide having an amino acid sequence of SEQ ID NO: 7;
      (g2) a polypeptide having an amino acid sequence in which one or more amino acids are deleted, substituted, or added in the amino acid sequence of SEQ ID NO: 7, and having a function as the heavy chain variable region;
      (g3) a polypeptide having an amino acid sequence that has homology of 80% or higher with respect to the amino acid sequence of SEQ ID NO: 7, and having a function as the heavy chain variable region; and
   (h) a light chain variable region which is any of the following (h1) through (h3):
      (h1) a polypeptide having an amino acid sequence of SEQ ID NO: 8;
      (h2) a polypeptide having an amino acid sequence in which one or more amino acids are deleted, substituted, or added in the amino acid sequence of SEQ ID NO: 8, and having a function as the light chain variable region;
      (h3) a polypeptide having an amino acid sequence that has homology of 80% or higher with respect to the amino acid sequence of SEQ ID NO: 8, and having a function as the light chain variable region.
<7> The anti-integrin α11 monoclonal antibody described in any one of <1> through <4>, including:
   (a) a heavy chain variable region CDR1 which is any of the following (a1) through (a3):
      (a1) a polypeptide having an amino acid sequence of SEQ ID NO: 1;
      (a2) a polypeptide having an amino acid sequence in which one or more amino acids are deleted, substituted, or added in the amino acid sequence of SEQ ID NO: 1, and having a function as the heavy chain variable region CDR1;
      (a3) a polypeptide having an amino acid sequence that has homology of 80% or higher with respect to the amino acid sequence of SEQ ID NO: 1, and having a function as the heavy chain variable region CDR1;
   (b) a heavy chain variable region CDR2 which is any of the following (b1) through (b3):
      (b1) a polypeptide having an amino acid sequence of SEQ ID NO: 2;
      (b2) a polypeptide having an amino acid sequence in which one or more amino acids are deleted, substituted, or added in the amino acid sequence of SEQ ID NO: 2, and having a function as the heavy chain variable region CDR2;
      (b3) a polypeptide having an amino acid sequence that has homology of 80% or higher with respect to the amino acid sequence of SEQ ID NO: 2, and having a function as the heavy chain variable region CDR2;
   (c') a heavy chain variable region CDR3 which is a polypeptide having an amino acid sequence of the following Formula (1):

      G(X₁X₂X₃X₄)C(X₅X₆)GDAACIDA ... (1)

      wherein:
      X₁ is S, H, or E,
      X₂ is A, T, F, S, M, or E,
      X₃ is G, D, V or A,
      X₄ is W or D,
      X₅ is W,
      X₆ is M;
   (d) a light chain variable region CDR1 which is any of the following (d1) through (d3):
      (d1) a polypeptide having an amino acid sequence of SEQ ID NO: 4;
      (d2) a polypeptide having an amino acid sequence in which one or more amino acids are deleted, substituted, or added in the amino acid sequence of SEQ ID NO: 4, and having a function as the light chain variable region CDR1;
      (d3) a polypeptide having an amino acid sequence that has homology of 80% or higher with respect to the amino acid sequence of SEQ ID NO: 4, and having a function as the light chain variable region CDR1;
   (e) a light chain variable region CDR2 which is any of the following (e1) through (e3):
      (e1) a polypeptide having an amino acid sequence of SEQ ID NO: 5;
      (e2) a polypeptide having an amino acid sequence in which one or more amino acids are deleted, substituted, or added in the amino acid sequence of SEQ ID NO: 5, and having a function as the light chain variable region CDR2;
      (e3) a polypeptide having an amino acid sequence that has homology of 80% or higher with respect to the amino acid sequence of SEQ ID NO: 5, and having a function as the light chain variable region CDR2; and
   (f) a light chain variable region CDR3 which is any of the following (f1) through (f3):
      (f1) a polypeptide having an amino acid sequence of SEQ ID NO: 6;
      (f2) a polypeptide having an amino acid sequence in which one or more amino acids are deleted, substituted, or added in the amino acid sequence of SEQ ID NO: 6, and having a function as the light chain variable region CDR3;
      (f3) a polypeptide having an amino acid sequence that has homology of 80% or higher with respect to the amino acid sequence of SEQ ID NO: 6, and having a function as the light chain variable region CDR3.
<8> A polynucleotide including a base sequence which encodes an anti-integrin α11 monoclonal antibody described in any one of <5> through <7>.
<9> A vector including a polynucleotide described in <8>.
<10> A treatment agent for a fibrotic disease, the treatment agent including: an anti-integrin α11 monoclonal antibody described in any one of <1> through <7> as an active ingredient; a polynucleotide described in <8> as an active ingredient; or a vector described in <9> as an active ingredient.
<11> The treatment agent described in <10>, in which the fibrotic disease is at least one disease selected from the group consisting of pulmonary fibrosis, hepatic fibrosis, nephrosclerosis, cardiomyopathy, cirrhosis, pancreatic fibrosis, and scleroderma.
<12> Use of an anti-integrin α11 monoclonal antibody described in any one of <1> through <7>, a polynucleotide described in <8>, or a vector described in <9> for the manufacture of a treatment agent for a fibrotic disease.
<13> A diagnostic agent for a fibrotic disease, the diagnostic agent including: an anti-integrin α11 monoclonal antibody described in any one of <1> through <7>; a polynucleotide described in <8>; or a vector described in <9>.

### Advantageous Effects of Invention

According to an aspect of the present invention, a novel measure for treating a fibrotic disease can be provided.

### Brief Description of Drawings

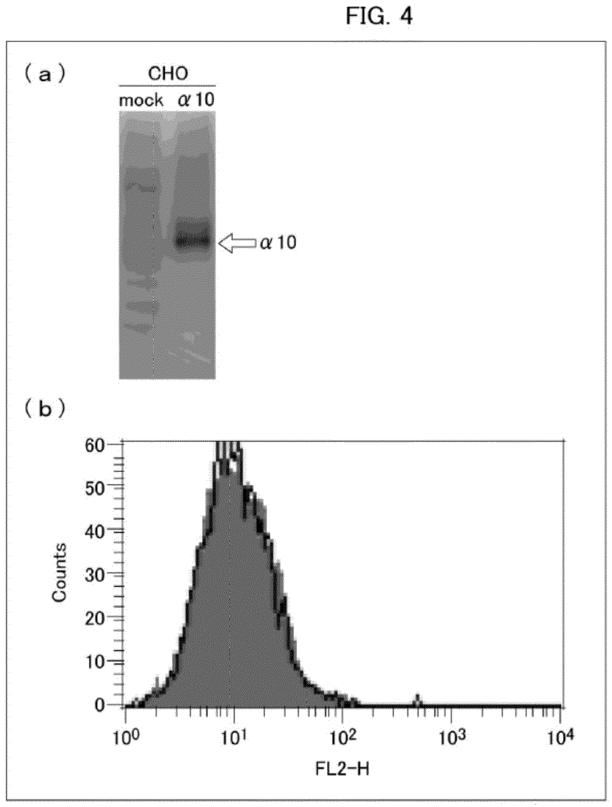

Fig. 1 is a diagram showing an amino acid sequence of a variable region of an antibody in accordance with an aspect of the present invention.
Fig. 2 is a diagram showing results of analyzing, by FACS, reactivity of the antibody in accordance with an aspect of the present invention with respect to an integrin α11. (a) of Fig. 2 shows a result of a case where a CHO cell (control) was used and (b) of Fig. 2 shows a result of a case where a human integrin α11 introduced CHO cell was used.
   (a) of Fig. 3 is a diagram showing a result of confirming, by FACS, that an SW480 cell expresses integrins α1 and α2.
   (b) of Fig. 3 is a diagram showing a result of analyzing, by FACS using an SW480 cell, nonreactivity of the antibody in accordance with an aspect of the present invention with respect to integrins α1 and α2.
   (a) of Fig. 4 is a diagram showing a result of confirming, by western blotting, that a human integrin α10 introduced CHO cell expresses an integrin α10. (b) of Fig. 4 is a diagram showing a result of analyzing, by FACS using a human integrin α10 introduced CHO cell, nonreactivity of the antibody in accordance with an aspect of the present invention with respect to an integrin α10.

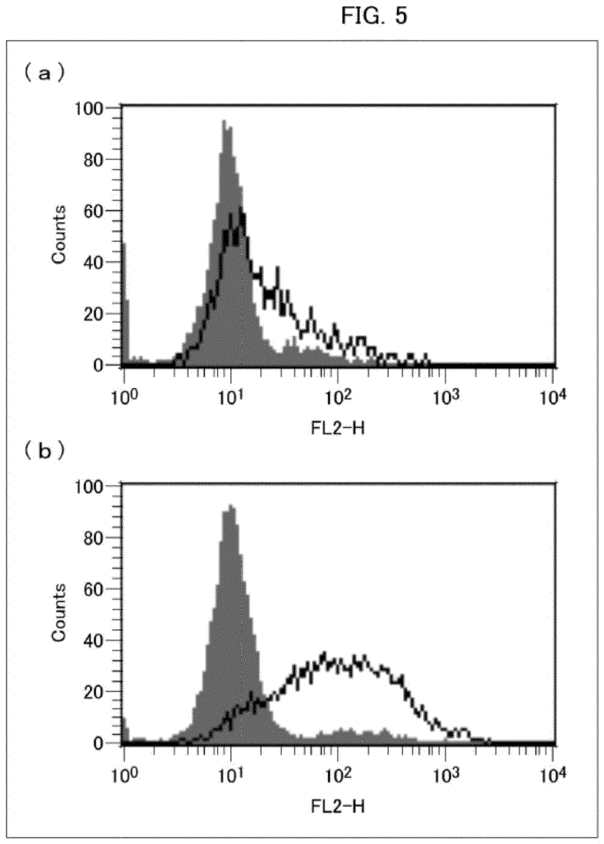

Fig. 5 is a diagram showing a result of analyzing, by FACS, reactivity of the antibody in accordance with an aspect of the present invention with respect to a rat hepatic stellate cell. (a) of Fig. 5 shows a result on day 5 of culture and (b) of Fig. 5 shows a result on day 10 of culture.

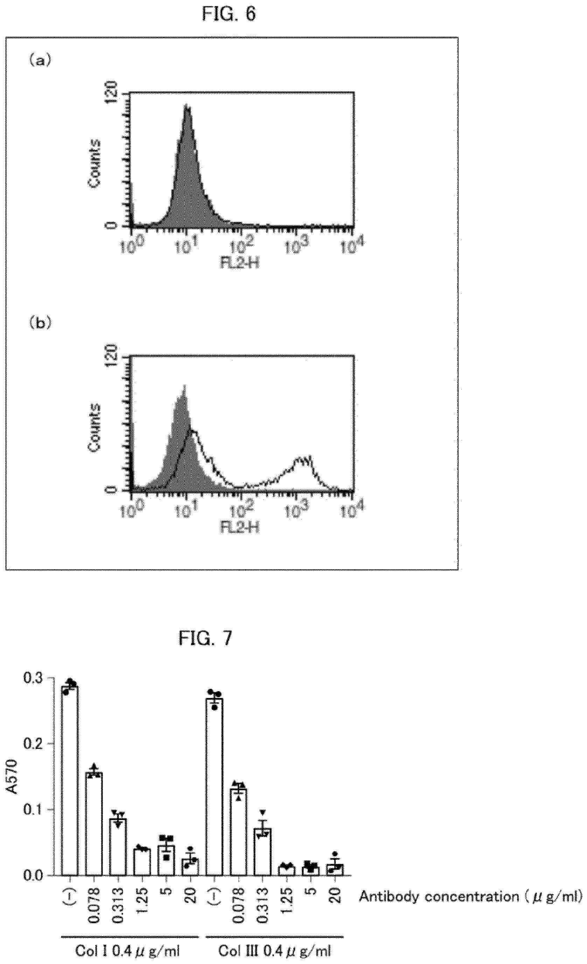

Fig. 6 is a diagram showing a result of analyzing, by FACS, reactivity of the antibody in accordance with an aspect of the present invention with respect to a rat integrin α11 introduced CHO cell. (a) of Fig. 6 shows reactivity of the antibody with respect to a CHO cell (control), and (b) of Fig. 6 shows reactivity of the antibody with respect to the rat integrin α11 introduced CHO cell.
Fig. 7 is a diagram showing a result of analyzing cell adhesion inhibitory activity of the antibody in accordance with an aspect of the present invention.

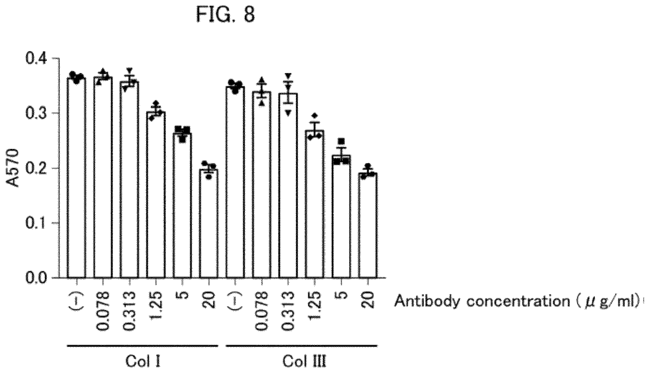

Fig. 8 is a diagram showing a result of analyzing cell detachment activity of the antibody in accordance with an aspect of the present invention.

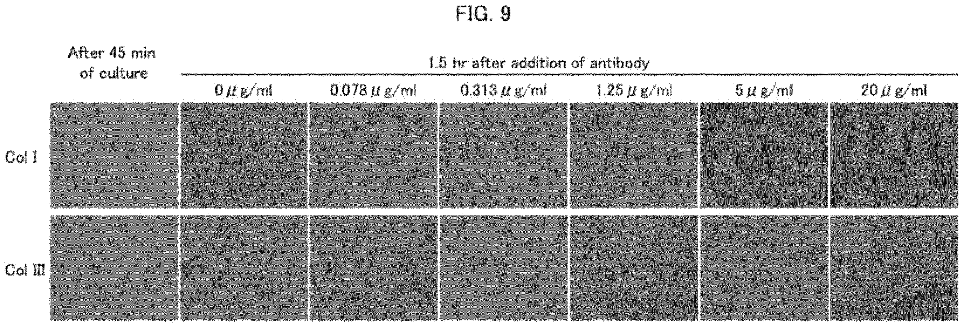

Fig. 9 is a diagram showing a result of analyzing cell spreading inhibitory activity of the antibody in accordance with an aspect of the present invention.

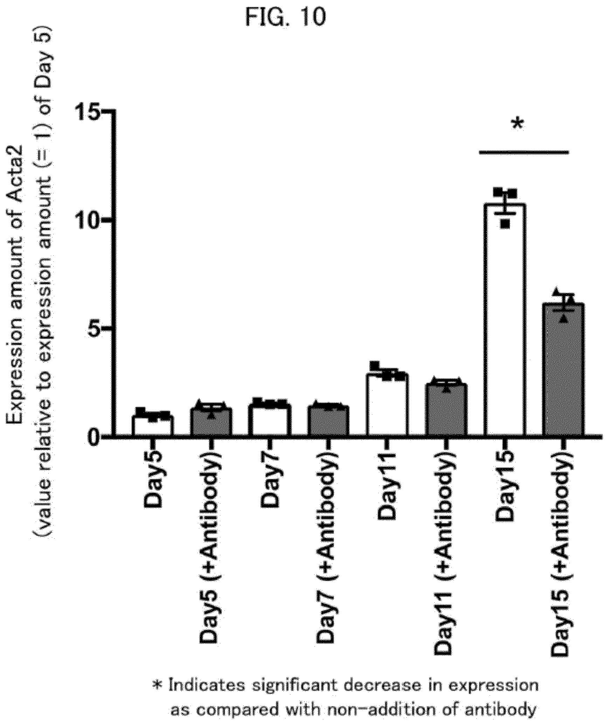

Fig. 10 is a diagram showing a result of a myofibroblast differentiation inhibition test of the antibody in accordance with an aspect of the present invention (6. in Examples (Experiment 1) described later).

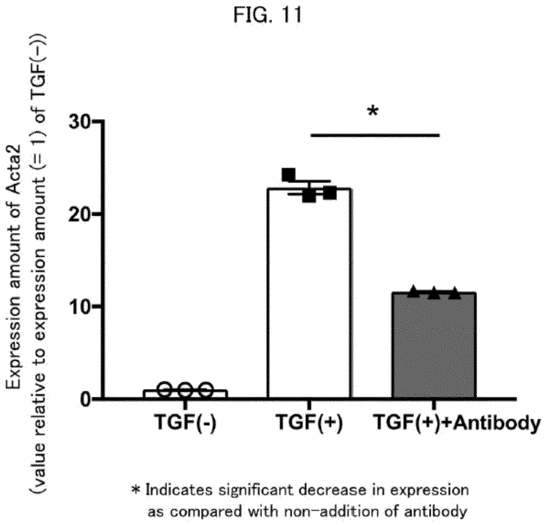

Fig. 11 is a diagram showing a result of a myofibroblast differentiation inhibition test of the antibody in accordance with an aspect of the present invention (6. in Examples (Experiment 2) described later).

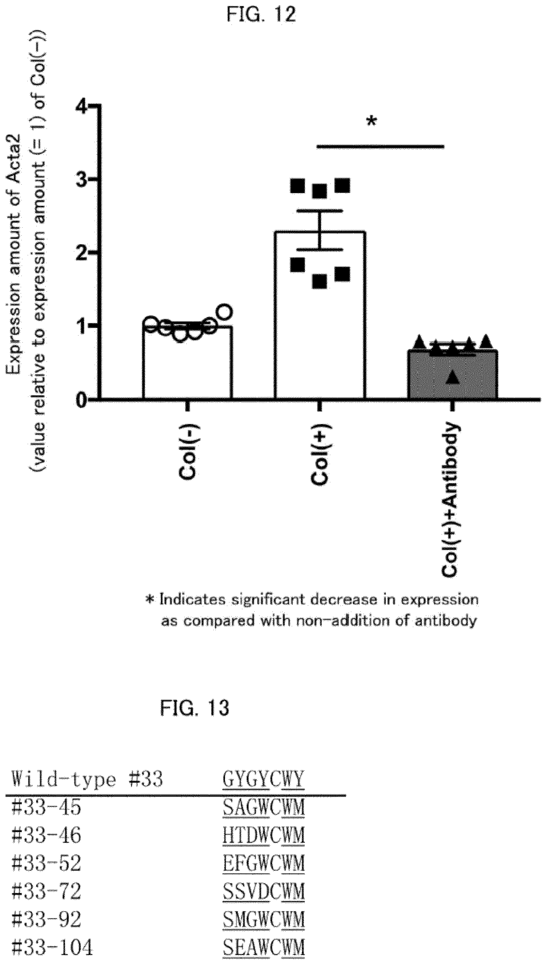

Fig. 12 is a diagram showing a result of a myofibroblast differentiation inhibition test of the antibody in accordance with an aspect of the present invention (6. in Examples (Experiment 3) described later).
Fig. 13 is a diagram showing an amino acid sequence of a part in a heavy chain variable region CDR3 of each of the antibody and modified antibodies in accordance with an aspect of the present invention. In Fig. 13, underlined parts indicate hot spots.

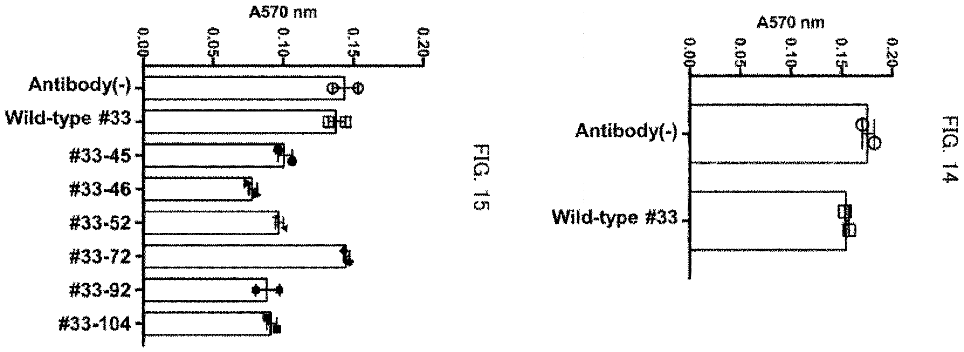

Fig. 14 is a diagram showing a result of a cell adhesion inhibition test of the antibody in accordance with an aspect of the present invention at a specific collagen concentration (4. in Examples described later).
Fig. 15 is a diagram showing a result of a cell adhesion inhibition test of the antibody in accordance with an aspect of the present invention at a specific collagen concentration (4. in Examples described later).

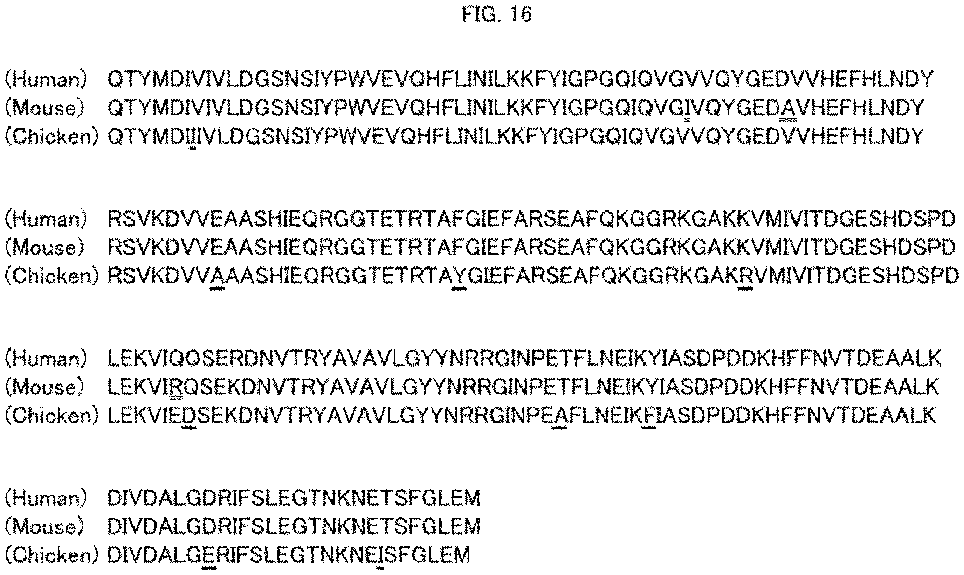

Fig. 16 is a diagram showing amino acid sequences of I-domain proteins of integrins α11 in a human, a mouse, and a chicken.

### Description of Embodiments

The following description will discuss an example of an embodiment of the present invention in detail. The present invention is, however, not limited to the embodiment.

Numerical expressions such as "A to B" herein mean "not less than A and not more than B" unless otherwise stated.

In this specification, "gene" is intended to be a polymer of nucleotides and is used synonymously with "polynucleotide", "nucleic acid", or "nucleic acid molecule". The gene can exist in a form of DNA (e.g., cDNA or genomic DNA) or in a form of RNA (e.g., mRNA). The DNA or RNA can be double-stranded or single-stranded. The single-stranded DNA or RNA can be a coding strand (sense strand) or a noncoding strand (antisense strand). The gene can be chemically synthesized. In addition, in a case where "polynucleotide" is described in this specification, the term can indicate DNA or RNA.

In this specification, "protein" is used synonymously with "peptide" or "polypeptide".

In this specification, bases and amino acids are represented with use of one-letter notation or three-letter notation as appropriate in accordance with rules provided for in IUPAC and IUB.

### [1. Antibody]

An antibody in accordance with an embodiment of the present invention is an anti-integrin α11 monoclonal antibody (hereinafter referred to as "antibody of an aspect of the present invention") which inhibits binding between an integrin α11 and collagen.

Integrin is a receptor that exists in a cell membrane surface as a heterodimer which is generally constituted by an α-chain and a β-chain. Integrin has been reported to function primarily as a receptor of an extracellular matrix. It is known that there are 18 α-chains and 8 β-chains as subunits, and that there are 24 types of integrin in total.

Among those types of integrin, an integrin α11, which is a target molecule of the antibody of an aspect of the present invention, is an integrin forming a heterodimer with β1, and has binding activity with collagen. Overexpression of the integrin α11 promotes tissue fibrosis. Therefore, the integrin α11 has been thought to be involved in fibrotic diseases (e.g., tissue fibrosis, tumor, and the like). So far, many researchers have attempted to obtain a monoclonal antibody with respect to an integrin α11 in order to develop an antibody drug for a fibrotic disease. However, despite many such studies, the monoclonal antibody with respect to an integrin α11 has not yet been obtained. A reason for this is that antibody production in which an animal is employed as a host uses a foreign protein as an antigen and is based on antigenicity resulting from a difference from a host protein. However, α11 is well conserved among mammals (that is, amino acid sequences of those are high in homology), and immunization of a mouse, which is a general-purpose host, with human α11 is likely to induce poor reaction as a foreign substance. The same seems to apply to a rat and a rabbit. However, in a case where a chicken is used as a host as in the present invention, human or mouse α11 seemed to have high antigenicity with respect to the chicken because a protein of α11 is greatly different between a bird and a mammal.

The inventors of the present invention carried out diligent studies in order to obtain an anti-integrin α11 monoclonal antibody. As a result, the anti-integrin α11 monoclonal antibody has been successfully obtained for the first time by the following procedures: (i) immunizing a chicken with use of an I-domain protein of mouse integrin α11 (Fig. 16); (ii) preparing an scFv phage antibody library on the basis of RNA derived from a lymphocyte obtained from a chicken spleen after immunization; and (iii) carrying out screening.

The antibody of an aspect of the present invention has the following characteristics:
- Having activity to inhibit binding between an integrin α11 and collagen (see 4. in Examples).
- Having cell adhesion inhibitory activity (see 4. in Examples).
- Having cell detachment activity (see 5. in Examples).
- Having cell spreading inhibitory activity (see 5. in Examples).
- Having tissue fibrosis inhibitory activity (see 6. in Examples).
- Specifically binding to an integrin α11 (see 2. in Examples).
- Binding to integrins α11 derived from two or more different species of mammals (see 3. in Examples).

Among the above characteristics, particularly for the cell detachment activity, there have been few reports on antibodies having that activity. In addition, a mechanism of the cell detachment activity has hardly been elucidated. Under such circumstances, it is surprising that the inventors of the present invention have succeeded in obtaining the anti-integrin α11 monoclonal antibody and that the antibody has the activity described above. From these, the antibody of an aspect of the present invention, which has the above effect, is expected to be extremely effective in treatment of a fibrotic disease and the like.

In addition, the inventors of the present invention carried out detailed studies of the antibody of an aspect of the present invention obtained above from the viewpoint of affinity. Specifically, a heavy chain variable region CDR3 is considered to be of utmost importance for affinity determination of the antibody. It is known that the antibody has a hot spot which is a site that is frequently mutated during affinity maturation. An example is reported in which a modified antibody having high affinity was produced by introducing a mutation into a hot spot site in vitro (Chowdhury PS et alo, Nat. Biotechnol., 1999 Jun; 17(6): 568-72).

Under the circumstances, the heavy chain variable region CDR3 of the antibody of an aspect of the present invention obtained above was variously modified and analyzed, and consequently antibodies having higher cell adhesion inhibitory activity than that of the above antibody have been successfully obtained. Thus, the antibodies in which the heavy chain variable region CDR3 has been modified and which have the higher cell adhesion inhibitory activity are also included in the antibody of an aspect of the present invention.

In this specification, "antibody" means a molecule which is capable of specifically binding to a particular epitope on an antigen.

In this specification, "monoclonal antibody" means an antibody obtained from a group of substantially uniform antibodies. That is, "monoclonal antibody" includes an antibody obtained from a group of identical antibodies, except for mutations that can be present in only a small amount and can naturally occur. The monoclonal antibody is highly specific, and each monoclonal antibody corresponds to a single epitope of antigen. A method for producing a monoclonal antibody is not particularly limited, and any method in this technical field can be used. For example, the antibody of an aspect of the present invention can be produced by a hybridoma method (Kohler G, Milstein C., Nature. 1975 Aug 7; 256(5517): 495-497), a recombinant method (US Patent No. 4816567), isolation from a phage antibody library (Clackson et al., Nature. 1991 Aug 15; 352 (6336): 624-628 or Marks et al., J Mol Biol. 1991 Dec 5; 222(3): 581-597), a general method for producing a monoclonal antibody ("Protein Experiment Handbook", YODOSHA CO., LTD (2003): 92-96), and the like. The antibody of an aspect of the present invention is preferably produced by a method in Examples described later.

The antibody of an aspect of the present invention encompasses a variety of forms and can be, for example, a low molecular antibody obtained with use of a fragment of antibody, as well as a full-length antibody. Examples of the low molecular antibody include Fv, Fab, F(ab')₂, Fab', diabody, a single-stranded antibody (e.g., scFv, dsFv), a CDR-containing peptide, a polyvalent specific antibody (e.g., bivalent specific antibody), and the like. The antibody of an aspect of the present invention can also be a mouse-chimeric antibody, a chicken-chimeric antibody, a humanized antibody, or the like. Furthermore, the antibody of an aspect of the present invention can be a form of low molecular antibody bound to a pre-existing chemosynthetic medicine progenitor or to a pharmaceutical formulation, or can be a form of sugar chain modified antibody. The various forms of antibody are disclosed, for example, in "Antibodies - A laboratory Manual -, Edward A. Greenfield Ed, Cold Spring Harbor Laboratory Press, NY", and the like.

Note that the antibody of an aspect of the present invention can be appropriately selected from the above various forms in order to reduce immunogenicity or to enhance stability when used as a therapeutic agent. For example, in order to reduce immunogenicity, the antibody of an aspect of the present invention is preferably used in a form of a chimeric antibody, a humanized antibody, or the like.

The low molecular antibody as described above can be produced by a known cloning technology or a known chemosynthetic method. For example, a cloning technology can be used to (i) prepare DNA which encodes the antibody fragment (low molecular antibody) described above, (ii) insert the DNA into an autonomously replicable vector to form recombinant DNA, (iii) introduce the recombinant DNA into a host such as E. coli, Bacillus subtilis, actinomyces, yeast, filamentous fungus, a plant cell, an insect cell, or an animal cell as appropriate to form a transformant, and (iv) obtain a peptide containing the amino acid sequence from the cultured matter (see, for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

Further, it is possible to obtain the low molecular antibody described above by (i) preparing DNA which encodes the low molecular antibody and (ii) carrying out synthesis by a cell-free protein synthesis system using the DNA and a wheat germ or an E. coli cell extract or the like. Alternatively, it is possible to obtain the above low molecular antibody by sequentially dehydrating and condensing and extending the amino acid with use of a conventional peptide chemosynthetic method such as "solid phase method" or "liquid phase method".

The antibody of an aspect of the present invention has at least one activity selected from the group consisting of cell adhesion inhibitory activity and cell detachment activity.

In this specification, "cell adhesion inhibitory activity" means activity that inhibits binding between collagen and cells having an integrin α11. That is, the antibody of an aspect of the present invention has activity that inhibits binding between collagen and a cell having an integrin α11 which is not being bound. Adhesion inhibition is a phenomenon that occurs because an integrin α11β1 formed by α11 cannot be bound to a ligand. The adhesion inhibition reflects a loss of intracellular signaling derived from α11β1 caused by ligand binding. The antibody of an aspect of the present invention, which has cell adhesion inhibitory activity, can prevent tissue fibrosis and inhibit progression of fibrosis. Measurement of cell adhesion inhibitory activity is not particularly limited and can be carried out using any of methods in this field. Cell adhesion inhibitory activity can be measured, for example, by a method in Examples described later. The cell adhesion inhibitory activity can also be assessed based on cell spreading inhibitory activity or tissue fibrosis inhibitory activity which are obtained as a result of cell adhesion inhibition.

In this specification, "cell detachment activity" means activity that dissociates a cell having an integrin α11 from collagen. That is, the antibody of an aspect of the present invention has activity that dissociates collagen from an integrin α11β1 which is being bound. The antibody of an aspect of the present invention, which has the cell detachment activity, can treat a fibrotic tissue. Measurement of cell detachment activity is not particularly limited and can be carried out using any of methods in this field. Cell detachment activity can be measured, for example, by a method in Examples described later.

Until now, a mechanism of the cell detachment activity has not been directly proved by crystal analysis or the like. However, whether an epitope and a ligand binding site of the inhibitory antibody at least partially overlap with each other (competitive) or are slightly distant from each other (allosteric) on an integrin α subunit is thought to be involved in the mechanism of cell detachment activity (see Mould AP, Biochem J., 464(3): 301-13, 2014, Mould AP, J Biol Chem., 291: 20993-1007, 2016).

The antibody of an aspect of the present invention specifically binds to an integrin α11.

In this specification, the term "specifically bind" means binding only to a target and not to others. The term "specifically bind" also includes preferentially binding only to a target. As shown in Examples described later, the antibody of an aspect of the present invention binds to an integrin α11 but does not exhibit binding activity to an integrin α1, an integrin α2, and an integrin α10, which are structurally similar to an integrin α11. Thus, the antibody of an aspect of the present invention is considered to have specificity with respect to an integrin α11.

The antibody of an aspect of the present invention specifically binds to integrins α11 derived from two or more different species of mammals.

The antibody of an aspect of the present invention has been demonstrated to bind to integrins α11 derived from two or more different species of mammals (in Examples, a human integrin α11 and a rat integrin α11) in Examples described later. Therefore, it is possible to obtain an anti-integrin α11 monoclonal antibody having cross-reactivity with respect to integrins α11 derived from two or more different species of mammals.

In this specification, "cross-reactivity" is a generic term indicative of a property in which a certain antibody has significant binding affinity with respect to two or more kinds of antigens having similar structures. Here, the antigens having similar structures include proteins having higher homology.

The antibody of an aspect of the present invention exhibits cross-reactivity with respect to α11 of, for example, a human, a mouse, a guinea pig, a hamster, a rat, a mouse, a rabbit, a pig, a sheep, a goat, a cattle, a horse, a cat, a dog, a marmoset, a monkey, a chimpanzee, and the like, and preferably exhibits cross-reactivity with respect to α11 of a human, a mouse, a rat, an a monkey, more preferably a human and a mouse.

The antibody of an aspect of the present invention can bind to two or more different species of mammals. Therefore, the antibody of an aspect of the present invention can be suitably used as a treatment agent or the like for used in a pre-clinical test which is required to verify an effect on two or more species of mammals.

The antibody of an aspect of the present invention is the following antibody.

The anti-integrin α11 monoclonal antibody, including:
(a) a heavy chain variable region CDR1 which is any of the following (a1) through (a3):
   (a1) a polypeptide having an amino acid sequence of SEQ ID NO: 1;
   (a2) a polypeptide having an amino acid sequence in which one or more amino acids are deleted, substituted, or added in the amino acid sequence of SEQ ID NO: 1, and having a function as the heavy chain variable region CDR1;
   (a3) a polypeptide having an amino acid sequence that has homology of 80% or higher with respect to the amino acid sequence of SEQ ID NO: 1, and having a function as the heavy chain variable region CDR1;
(b) a heavy chain variable region CDR2 which is any of the following (b1) through (b3):
   (b1) a polypeptide having an amino acid sequence of SEQ ID NO: 2;
   (b2) a polypeptide having an amino acid sequence in which one or more amino acids are deleted, substituted, or added in the amino acid sequence of SEQ ID NO: 2, and having a function as the heavy chain variable region CDR2;
   (b3) a polypeptide having an amino acid sequence that has homology of 80% or higher with respect to the amino acid sequence of SEQ ID NO: 2, and having a function as the heavy chain variable region CDR2;
(c) a heavy chain variable region CDR3 which is any of the following (c1) through (c3):
   (c1) a polypeptide having an amino acid sequence of SEQ ID NO: 3;
   (c2) a polypeptide having an amino acid sequence in which one or more amino acids are deleted, substituted, or added in the amino acid sequence of SEQ ID NO: 3, and having a function as the heavy chain variable region CDR3;
   (c3) a polypeptide having an amino acid sequence that has homology of 80% or higher with respect to the amino acid sequence of SEQ ID NO: 3, and having a function as the heavy chain variable region CDR3;
(d) a light chain variable region CDR1 which is any of the following (d1) through (d3):
   (d1) a polypeptide having an amino acid sequence of SEQ ID NO: 4;
   (d2) a polypeptide having an amino acid sequence in which one or more amino acids are deleted, substituted, or added in the amino acid sequence of SEQ ID NO: 4, and having a function as the light chain variable region CDR1;
   (d3) a polypeptide having an amino acid sequence that has homology of 80% or higher with respect to the amino acid sequence of SEQ ID NO: 4, and having a function as the light chain variable region CDR1;
(e) a light chain variable region CDR2 which is any of the following (e1) through (e3):
   (e1) a polypeptide having an amino acid sequence of SEQ ID NO: 5;
   (e2) a polypeptide having an amino acid sequence in which one or more amino acids are deleted, substituted, or added in the amino acid sequence of SEQ ID NO: 5, and having a function as the light chain variable region CDR2;
   (e3) a polypeptide having an amino acid sequence that has homology of 80% or higher with respect to the amino acid sequence of SEQ ID NO: 5, and having a function as the light chain variable region CDR2; and
(f) a light chain variable region CDR3 which is any of the following (f1) through (f3):
   (f1) a polypeptide having an amino acid sequence of SEQ ID NO: 6;
   (f2) a polypeptide having an amino acid sequence in which one or more amino acids are deleted, substituted, or added in the amino acid sequence of SEQ ID NO: 6, and having a function as the light chain variable region CDR3;
   (f3) a polypeptide having an amino acid sequence that has homology of 80% or higher with respect to the amino acid sequence of SEQ ID NO: 6, and having a function as the light chain variable region CDR3.

The polypeptide of the above (a) will be specifically described. The polypeptide of (a) above constitutes a heavy chain variable region CDR1 in the antibody of an aspect of the present invention.

SEQ ID NO: 1 of (a1) above is a polypeptide derived from a chicken and is constituted by 5 amino acid residues in total length.

The polypeptide of (a2) is a functionally equivalent mutant, a derivative, a variant, an allele, a homologue, an orthologue, or a partial polypeptide of a protein having the amino acid sequence of SEQ ID NO: 1, a fusion protein with another protein/peptide, or the like, and the polypeptide is not limited in its specific sequence, provided that the polypeptide has a function as the heavy chain variable region CDR1. Here, the number of amino acids which can be deleted, substituted or added is not limited, provided that the foregoing function is not lost. The number refers to the number that can be deleted, substituted or added by a known introduction method such as a site-directed mutagenesis method, and is preferably within 3 amino acids (e.g., 3, 2 or 1 amino acid). In this specification, "mutation" mainly means a mutation artificially introduced by the site-directed mutagenesis method or the like. Note, however, that the mutation can be a similar mutation that occurs naturally.

It is preferable that the mutating amino acid residue is mutated into another amino acid in which properties of an amino acid side chain are conserved. For example, the properties of the amino acid side chain include the following properties: a hydrophobic amino acid (A, I, L, M, F, P, W, Y, V); a hydrophilic amino acid (R, D, N, C, E, Q, G, H, K, S, T); an amino acid having an aliphatic side chain (G, A, V, L, I, P); an amino acid having a hydroxyl-group-containing side chain (S, T, Y); an amino acid having sulfur-atom-containing side chain (C, M); an amino acid having a carboxylic acid and an amide-containing side chain (D, N, E, Q); an amino acid having a base-containing side chain (R, K, H); and an amino acid having an aromatic-group-containing side chain (H, F, Y, W) (letters in parentheses represent single-letter notations of respective amino acids). It is already known that a polypeptide having an amino acid sequence in which one or more amino acid residues have been modified with respect to a certain amino acid sequence by deletion, addition and/or substitution with other amino acid(s) maintains its biological activity. In addition, it is more preferable that a target amino acid residue is mutated into an amino acid residue having as many common properties as possible.

The phrase "functionally equivalent" in this specification intends that a subject polypeptide has a biological function and/or a biochemical function equivalent (identical and/or similar) to a polypeptide of interest. The biological properties can also include specificity of an expressed site, an expression level, and the like. Whether or not the mutated polypeptide has an intended function can be determined by checking whether or not the mutant polypeptide functions as the heavy chain variable region CDR1.

Whether or not the polypeptide is a polypeptide having a function as the heavy chain variable region CDR1 can be determined by testing whether or not an antibody containing that polypeptide and the polypeptides of (b) through (f) above is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the heavy chain variable region CDR1. A specific determination method is not particularly limited, and any method known in this field can be used.

The polypeptide of (a3) is also intended to be a functionally equivalent mutant, a derivative, a variant, an allele, a homologue, an orthologue, or a partial peptide of a protein having the amino acid sequence of SEQ ID NO: 1, a fusion protein with another protein/peptide, or the like, and the polypeptide is not limited in its specific sequence, provided that the polypeptide has a function as the heavy chain variable region CDR1. Homology of amino acid sequence means to have sequence homology of at least 80% or higher, more preferably 90% or higher, even more preferably 95% or higher (e.g., 95%, 96%, 97%, 98%, 99% or higher) in the entire amino acid sequence (or a region necessary for functional expression). The homology of amino acid sequence can be determined by utilizing a program of BLASTN (nucleic acid level) or BLASTX (amino acid level) (Altschul et al. J. Mol. Biol., 215: 403-410, 1990). The program is based on an algorithm BLAST by Karlin and Altschul (Proc. Natl. Acad. Sci. USA, 87: 2264-2268, 1990, Proc. Natl. Acad. Sci. USA, 90: 5873-5877, 1993). In a case where a base sequence is analyzed by BLASTN, parameters are set to, for example, score = 100 and wordlength = 12. In a case where an amino acid sequence is analyzed by BLASTX, parameters are set to, for example, score = 50 and wordlength = 3. In a case where an amino acid sequence is analyzed by using Gapped BLAST program, the analysis can be carried out as described in Altschul et al. (Nucleic Acids Res. 25: 3389-3402, 1997). In a case where BLAST and Gapped BLAST programs are used, default parameters of the programs are used. Specific procedures of those analysis methods are known. Addition or deletion (e.g., a gap or the like) can be accepted in order to optimally align base sequences or amino acid sequences to be compared.

In this specification, "homology" is intended to be a ratio of the number of amino acid residues having similar properties (positive or the like). More preferably, "homology" is a ratio of the number of identical amino acid residues, that is, identity. Note that the properties of the amino acid are as described above.

The polypeptide in accordance with an aspect of the present invention can be any polypeptide in which amino acids are peptide-bonded, but is not limited to this. That is, the polypeptide of an aspect of the present invention can be a composite peptide having a structure other than peptide, e.g., a sugar chain, an isoprenoid group, or the like. A functional group of the amino acid can be modified. The amino acid is preferably L-type, but is not limited to this.

As a method of obtaining the above polypeptide, a polynucleotide modification method which is usually carried out can be used. That is, the polynucleotide having genetic information of an intended recombinant protein can be prepared by substituting, deleting, inserting and/or adding a particular base of a polynucleotide having genetic information of a protein. A specific method of converting a base of a polynucleotide can be, for example, use of a commercially available kit (KOD-Plus Site-Directed Mutagenesis Kit; available from Toyobo Co., Ltd., Transformer Site-Directed Mutagenesis Kit; available from Clontech, QuickChange Site Directed Mutagenesis Kit; available from Stratagene, or the like), or use of a polymerase chain reaction (PCR) method. Those methods are known to those skilled in the art.

The polypeptide of an aspect of the present invention can be easily prepared in accordance with a publicly known arbitrary method in this field. For example, the polypeptide of an aspect of the present invention can be expressed by a transformant into which a polypeptide expression vector is introduced or can be chemically synthesized. The chemosynthetic method can be a solid phase method or a liquid phase method. In the solid phase method, for example, any of commercially available peptide synthesis devices (Model MultiPep RS (Intavis AG) or the like) can be used.

Next, the polypeptides in (b) through (f) will be described only in parts that differ from (a) above.

The polypeptide of (b) above constitutes a heavy chain variable region CDR2 in the antibody of an aspect of the present invention.

SEQ ID NO: 2 of (b1) above is a polypeptide derived from a chicken and is constituted by 17 amino acid residues in total length.

The polypeptide of (b2) is a functionally equivalent mutant, a derivative, a variant, an allele, a homologue, an orthologue, or a partial polypeptide of a protein having an amino acid sequence of SEQ ID NO: 2, a fusion protein with another protein/peptide, or the like, and the polypeptide is not limited in its specific sequence, provided that the polypeptide has a function as the heavy chain variable region CDR2. Whether or not the mutated polypeptide has an intended function can be determined by checking whether or not the mutant polypeptide functions as the heavy chain variable region CDR2.

Whether or not the polypeptide is a polypeptide having a function as the heavy chain variable region CDR2 can be determined by testing whether or not an antibody containing that polypeptide and the polypeptides of (a) and (c) through (f) above is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the heavy chain variable region CDR2.

The polypeptide of (b3) is also intended to be a functionally equivalent mutant, a derivative, a variant, an allele, a homologue, an orthologue, or a partial peptide of a protein having the amino acid sequence of SEQ ID NO: 2, a fusion protein with another protein/peptide, or the like, and the polypeptide is not limited in its specific sequence, provided that the polypeptide has a function as the heavy chain variable region CDR2.

The polypeptide of (c) above constitutes a heavy chain variable region CDR3 in the antibody of an aspect of the present invention.

SEQ ID NO: 3 of (c1) above is a polypeptide derived from a chicken and is constituted by 16 amino acid residues in total length.

The polypeptide of (c2) is a functionally equivalent mutant, a derivative, a variant, an allele, a homologue, an orthologue, or a partial polypeptide of a protein having an amino acid sequence of SEQ ID NO: 3, a fusion protein with another protein/peptide, or the like, and the polypeptide is not limited in its specific sequence, provided that the polypeptide has a function as the heavy chain variable region CDR3. Whether or not the mutated polypeptide has an intended function can be determined by checking whether or not the mutant polypeptide functions as the heavy chain variable region CDR3.

Whether or not the polypeptide is a polypeptide having a function as the heavy chain variable region CDR3 can be determined by testing whether or not an antibody containing that polypeptide and the polypeptides of (a), (b) and (d) through (f) above is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the heavy chain variable region CDR3.

The polypeptide of (c3) is also intended to be a functionally equivalent mutant, a derivative, a variant, an allele, a homologue, an orthologue, or a partial peptide of a protein having the amino acid sequence of SEQ ID NO: 3, a fusion protein with another protein/peptide, or the like, and the polypeptide is not limited in its specific sequence, provided that the polypeptide has a function as the heavy chain variable region CDR3.

The polypeptide of (d) above constitutes a light chain variable region CDR1 in the antibody of an aspect of the present invention.

SEQ ID NO: 4 of (d1) above is a polypeptide derived from a chicken and is constituted by 9 amino acid residues in total length.

The polypeptide of (d2) is a functionally equivalent mutant, a derivative, a variant, an allele, a homologue, an orthologue, or a partial polypeptide of a protein having an amino acid sequence of SEQ ID NO: 4, a fusion protein with another protein/peptide, or the like, and the polypeptide is not limited in its specific sequence, provided that the polypeptide has a function as the light chain variable region CDR1. Whether or not the mutated polypeptide has an intended function can be determined by checking whether or not the mutant polypeptide functions as the light chain variable region CDR1.

Whether or not the polypeptide is a polypeptide having a function as the light chain variable region CDR1 can be determined by testing whether or not an antibody containing that polypeptide and the polypeptides of (a) through (c) and (e) through (f) above is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the light chain variable region CDR1.

The polypeptide of (d3) is also intended to be a functionally equivalent mutant, a derivative, a variant, an allele, a homologue, an orthologue, or a partial peptide of a protein having the amino acid sequence of SEQ ID NO: 4, a fusion protein with another protein/peptide, or the like, and the polypeptide is not limited in its specific sequence, provided that the polypeptide has a function as the light chain variable region CDR1.

The polypeptide of (e) above constitutes a light chain variable region CDR2 in the antibody of an aspect of the present invention.

SEQ ID NO: 5 of (e1) above is a polypeptide derived from a chicken and is constituted by 7 amino acid residues in total length.

The polypeptide of (e2) is a functionally equivalent mutant, a derivative, a variant, an allele, a homologue, an orthologue, or a partial polypeptide of a protein having an amino acid sequence of SEQ ID NO: 5, a fusion protein with another protein/peptide, or the like, and the polypeptide is not limited in its specific sequence, provided that the polypeptide has a function as the light chain variable region CDR2. Whether or not the mutated polypeptide has an intended function can be determined by checking whether or not the mutant polypeptide functions as the light chain variable region CDR2.

Whether or not the polypeptide is a polypeptide having a function as the light chain variable region CDR2 can be determined by testing whether or not an antibody containing that polypeptide and the polypeptides of (a) through (d) and (f) above is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the light chain variable region CDR2.

The polypeptide of (e3) is also intended to be a functionally equivalent mutant, a derivative, a variant, an allele, a homologue, an orthologue, or a partial peptide of a protein having the amino acid sequence of SEQ ID NO: 5, a fusion protein with another protein/peptide, or the like, and the polypeptide is not limited in its specific sequence, provided that the polypeptide has a function as the light chain variable region CDR2.

The polypeptide of (f) above constitutes a light chain variable region CDR3 in the antibody of an aspect of the present invention.

SEQ ID NO: 6 of (f1) above is a polypeptide derived from a chicken and is constituted by 10 amino acid residues in total length.

The polypeptide of (f2) is a functionally equivalent mutant, a derivative, a variant, an allele, a homologue, an orthologue, or a partial polypeptide of a protein having an amino acid sequence of SEQ ID NO: 6, a fusion protein with another protein/peptide, or the like, and the polypeptide is not limited in its specific sequence, provided that the polypeptide has a function as the light chain variable region CDR3. Whether or not the mutated polypeptide has an intended function can be determined by checking whether or not the mutant polypeptide functions as the light chain variable region CDR3.

Whether or not the polypeptide is a polypeptide having a function as the light chain variable region CDR3 can be determined by testing whether or not an antibody containing that polypeptide and the polypeptides of (a) through (e) above is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the light chain variable region CDR3.

The polypeptide of (f3) is also intended to be a functionally equivalent mutant, a derivative, a variant, an allele, a homologue, an orthologue, or a partial peptide of a protein having the amino acid sequence of SEQ ID NO: 6, a fusion protein with another protein/peptide, or the like, and the polypeptide is not limited in its specific sequence, provided that the polypeptide has a function as the light chain variable region CDR3.

The antibody of an aspect of the present invention is the following antibody.

The anti-integrin α11 monoclonal antibody, including:
(g) a heavy chain variable region which is any of the following (g1) through (g3):
   (g1) a polypeptide having an amino acid sequence of SEQ ID NO: 7;
   (g2) a polypeptide having an amino acid sequence in which one or more amino acids are deleted, substituted, or added in the amino acid sequence of SEQ ID NO: 7, and having a function as the heavy chain variable region;
   (g3) a polypeptide having an amino acid sequence that has homology of 80% or higher with respect to the amino acid sequence of SEQ ID NO: 7, and having a function as the heavy chain variable region;
(h) a light chain variable region which is any of the following (h1) through (h3):
   (h1) a polypeptide having an amino acid sequence of SEQ ID NO: 8;
   (h2) a polypeptide having an amino acid sequence in which one or more amino acids are deleted, substituted, or added in the amino acid sequence of SEQ ID NO: 8, and having a function as the light chain variable region;
   (h3) a polypeptide having an amino acid sequence that has homology of 80% or higher with respect to the amino acid sequence of SEQ ID NO: 8, and having a function as the light chain variable region.

The polypeptides in (g) and (h) will be described below only in parts that differ from (a) above.

The polypeptide of (g) above constitutes a heavy chain variable region in the antibody of an aspect of the present invention.

SEQ ID NO: 7 of (g1) above is a polypeptide derived from a chicken and is constituted by 125 amino acid residues in total length.

The polypeptide of (g2) is a functionally equivalent mutant, a derivative, a variant, an allele, a homologue, an orthologue, or a partial polypeptide of a protein having an amino acid sequence of SEQ ID NO: 7, a fusion protein with another protein/peptide, or the like, and the polypeptide is not limited in its specific sequence, provided that the polypeptide has a function as the heavy chain variable region. Whether or not the mutated polypeptide has an intended function can be determined by checking whether or not the mutant polypeptide functions as the heavy chain variable region.

Whether or not the polypeptide is a polypeptide having a function as the heavy chain variable region can be determined by testing whether or not an antibody containing that polypeptide is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the heavy chain variable region.

The polypeptide of (g3) is also intended to be a functionally equivalent mutant, a derivative, a variant, an allele, a homologue, an orthologue, or a partial peptide of a protein having the amino acid sequence of SEQ ID NO: 7, a fusion protein with another protein/peptide, or the like, and the polypeptide is not limited in its specific sequence, provided that the polypeptide has a function as the heavy chain variable region.

The polypeptide of (h) above constitutes a light chain variable region in the antibody of an aspect of the present invention.

SEQ ID NO: 8 of (h1) above is a polypeptide derived from a chicken and is constituted by 105 amino acid residues in total length.

The polypeptide of (h2) is a functionally equivalent mutant, a derivative, a variant, an allele, a homologue, an orthologue, or a partial polypeptide of a protein having an amino acid sequence of SEQ ID NO: 8, a fusion protein with another protein/peptide, or the like, and the polypeptide is not limited in its specific sequence, provided that the polypeptide has a function as the light chain variable region. Whether or not the mutated polypeptide has an intended function can be determined by checking whether or not the mutant polypeptide functions as the light chain variable region.

Whether or not the polypeptide is a polypeptide having a function as the light chain variable region can be determined by testing whether or not an antibody containing that polypeptide is bound to an integrin α11 when the antibody is brought into contacted with the integrin α11 (or a sample containing the integrin α11). That is, if the antibody is bound to an integrin α11 when the antibody is brought into contact with the integrin α11 (or the sample containing the integrin α11), the polypeptide can be determined to be a polypeptide having a function as the light chain variable region.

The polypeptide of (h3) is also intended to be a functionally equivalent mutant, a derivative, a variant, an allele, a homologue, an orthologue, or a partial peptide of a protein having the amino acid sequence of SEQ ID NO: 8, a fusion protein with another protein/peptide, or the like, and the polypeptide is not limited in its specific sequence, provided that the polypeptide has a function as the light chain variable region.

The antibody of an aspect of the present invention is the following antibody.

The anti-integrin α11 monoclonal antibody, including:
(a) a heavy chain variable region CDR1 which is any of the following (a1) through (a3):
   (a1) a polypeptide having an amino acid sequence of SEQ ID NO: 1;
   (a2) a polypeptide having an amino acid sequence in which one or more amino acids are deleted, substituted, or added in the amino acid sequence of SEQ ID NO: 1, and having a function as the heavy chain variable region CDR1;
   (a3) a polypeptide having an amino acid sequence that has homology of 80% or higher with respect to the amino acid sequence of SEQ ID NO: 1, and having a function as the heavy chain variable region CDR1;
(b) a heavy chain variable region CDR2 which is any of the following (b1) through (b3):
   (b1) a polypeptide having an amino acid sequence of SEQ ID NO: 2;
   (b2) a polypeptide having an amino acid sequence in which one or more amino acids are deleted, substituted, or added in the amino acid sequence of SEQ ID NO: 2, and having a function as the heavy chain variable region CDR2;
   (b3) a polypeptide having an amino acid sequence that has homology of 80% or higher with respect to the amino acid sequence of SEQ ID NO: 2, and having a function as the heavy chain variable region CDR2;
(c') a heavy chain variable region CDR3 which is a polypeptide having an amino acid sequence of the following Formula (1):

   G(X₁X₂X₃X₄)C(X₅X₆)GDAACIDA ... (1)

   wherein:
   X₁ is S, H, or E,
   X₂ is A, T, F, S, M, or E,
   X₃ is G, D, V or A,
   X₄ is W or D,
   X₅ is W,
   X₆ is M;
(d) a light chain variable region CDR1 which is any of the following (d1) through (d3):
   (d1) a polypeptide having an amino acid sequence of SEQ ID NO: 4;
   (d2) a polypeptide having an amino acid sequence in which one or more amino acids are deleted, substituted, or added in the amino acid sequence of SEQ ID NO: 4, and having a function as the light chain variable region CDR1;
   (d3) a polypeptide having an amino acid sequence that has homology of 80% or higher with respect to the amino acid sequence of SEQ ID NO: 4, and having a function as the light chain variable region CDR1;
(e) a light chain variable region CDR2 which is any of the following (e1) through (e3):
   (e1) a polypeptide having an amino acid sequence of SEQ ID NO: 5;
   (e2) a polypeptide having an amino acid sequence in which one or more amino acids are deleted, substituted, or added in the amino acid sequence of SEQ ID NO: 5, and having a function as the light chain variable region CDR2;
   (e3) a polypeptide having an amino acid sequence that has homology of 80% or higher with respect to the amino acid sequence of SEQ ID NO: 5, and having a function as the light chain variable region CDR2; and
(f) a light chain variable region CDR3 which is any of the following (f1) through (f3):
   (f1) a polypeptide having an amino acid sequence of SEQ ID NO: 6;
   (f2) a polypeptide having an amino acid sequence in which one or more amino acids are deleted, substituted, or added in the amino acid sequence of SEQ ID NO: 6, and having a function as the light chain variable region CDR3;
   (f3) a polypeptide having an amino acid sequence that has homology of 80% or higher with respect to the amino acid sequence of SEQ ID NO: 6, and having a function as the light chain variable region CDR3.

That is, the antibody in this aspect is an antibody in which the sequence in the heavy chain variable region CDR3 of (c) above is optimized while using, as an indicator, various kinds of activity possessed by the antibody of an aspect of the present invention.

The polypeptide of (c') is obtained by introducing mutation into a hot spot site in the polypeptide of (c1) and by appropriately carrying out screening. For example, the polypeptide of (c') is obtained by a method in Examples described later.

The antibody in this aspect has particularly excellent effects in cell adhesion inhibitory activity and/or cell detachment activity, and the antibody is therefore extremely useful as a treatment agent or the like for a fibrotic disease described later.

In Formula (1) above, X₁, X₂, X₃, X₄, X₅, X₆ represent sequences important for enhancing the effect of the antibody of an aspect of the present invention. In Formula (1) above, X₁ can be S, H or E, X₂ can be A, T, F, S, M or E, X₃ can be G, D, V or A, X₄ can be W or D, X₅ can be W, and X₆ can be M. In Formula (1) above, X₄ is preferably W.

In an embodiment of the present invention, the heavy chain variable region CDR3 which is the polypeptide having the amino acid sequence represented by Formula (1) above is preferably a polypeptide having an amino acid sequence of any one of SEQ ID NOs: 57 through 62. That is, in an embodiment of the present invention, the antibody of an aspect of the present invention can be an antibody shown below.

The anti-integrin α11 monoclonal antibody, including:
(a) a heavy chain variable region CDR1 which is any of the following (a1) through (a3):
   (a1) a polypeptide having an amino acid sequence of SEQ ID NO: 1;
   (a2) a polypeptide having an amino acid sequence in which one or more amino acids are deleted, substituted, or added in the amino acid sequence of SEQ ID NO: 1, and having a function as the heavy chain variable region CDR1;
   (a3) a polypeptide having an amino acid sequence that has homology of 80% or higher with respect to the amino acid sequence of SEQ ID NO: 1, and having a function as the heavy chain variable region CDR1;
(b) a heavy chain variable region CDR2 which is any of the following (b1) through (b3):
   (b1) a polypeptide having an amino acid sequence of SEQ ID NO: 2;
   (b2) a polypeptide having an amino acid sequence in which one or more amino acids are deleted, substituted, or added in the amino acid sequence of SEQ ID NO: 2, and having a function as the heavy chain variable region CDR2;
   (b3) a polypeptide having an amino acid sequence that has homology of 80% or higher with respect to the amino acid sequence of SEQ ID NO: 2, and having a function as the heavy chain variable region CDR2;
(c") a heavy chain variable region CDR3 which is a polypeptide having an amino acid sequence of any of SEQ ID NOs: 57 through 62;
(d) a light chain variable region CDR1 which is any of the following (d1) through (d3):
   (d1) a polypeptide having an amino acid sequence of SEQ ID NO: 4;
   (d2) a polypeptide having an amino acid sequence in which one or more amino acids are deleted, substituted, or added in the amino acid sequence of SEQ ID NO: 4, and having a function as the light chain variable region CDR1;
   (d3) a polypeptide having an amino acid sequence that has homology of 80% or higher with respect to the amino acid sequence of SEQ ID NO: 4, and having a function as the light chain variable region CDR1;
(e) a light chain variable region CDR2 which is any of the following (e1) through (e3):
   (e1) a polypeptide having an amino acid sequence of SEQ ID NO: 5;
   (e2) a polypeptide having an amino acid sequence in which one or more amino acids are deleted, substituted, or added in the amino acid sequence of SEQ ID NO: 5, and having a function as the light chain variable region CDR2;
   (e3) a polypeptide having an amino acid sequence that has homology of 80% or higher with respect to the amino acid sequence of SEQ ID NO: 5, and having a function as the light chain variable region CDR2; and
(f) a light chain variable region CDR3 which is any of the following (f1) through (f3):
   (f1) a polypeptide having an amino acid sequence of SEQ ID NO: 6;
   (f2) a polypeptide having an amino acid sequence in which one or more amino acids are deleted, substituted, or added in the amino acid sequence of SEQ ID NO: 6, and having a function as the light chain variable region CDR3;
   (f3) a polypeptide having an amino acid sequence that has homology of 80% or higher with respect to the amino acid sequence of SEQ ID NO: 6, and having a function as the light chain variable region CDR3.

Moreover, in another embodiment of the present invention, the above (c") can be a heavy chain variable region CDR3 which is a polypeptide having an amino acid sequence of any of SEQ ID NOs: 57, 58, 59, 61 and 62.

Note that, in the above aspect including (c') or (c"), the polypeptides (i.e., polypeptides of (a), (b), (d) through (f)) other than (c') and (c") are as described above.

### [2. Polynucleotide]

The polynucleotide in accordance with an embodiment of the present invention (hereinafter referred to as "polynucleotide of an aspect of the present invention") includes a base sequence which encodes the anti-integrin α11 monoclonal antibody of 1. above.

The polynucleotide of an aspect of the present invention can be constituted only by the polynucleotide which encodes the antibody of 1. above or can additionally contain other base sequence(s). The additional base sequences are not particularly limited and examples thereof include base sequences which encode labels (e.g., a histidine tag, an Myc tag, a FLAG tag, and the like), fusion proteins (e.g., streptavidin, cytochrome, GST, GFP, MBP, and the like), and signal sequences (e.g., an endoplasmic reticulum translocation signal sequence, a secretory sequence, and the like). A site to which these base sequences are added is not particularly limited and can be, for example, an N-terminal or a C-terminal of a protein to be translated.

### [3. Vector]

The vector in accordance with an embodiment of the present invention (hereinafter referred to as "vector of an aspect of the present invention") includes the polynucleotide of 2. above.

The vector of an aspect of the present invention is not particularly limited and can be an expression vector or a cloning vector. The following description will discuss details of a case where the vector of an aspect of the present invention is an expression vector.

The vector of an aspect of the present invention can be any of various vectors commonly used as a substrate vector, and can be appropriately selected depending on a cell to be introduced or a method of introduction. Specifically, a plasmid, a phage, a cosmid, or the like can be used as the substrate vector. A specific type of the vector is not particularly limited, and a vector that can be expressed in a host cell can be appropriately selected.

Examples of the expression vector described above include a phage vector, a plasmid vector, a viral vector, a retroviral vector, a chromosomal vector, an episomal vector and virus-derived vectors (bacterial plasmid, bacteriophage, yeast episome, and the like), a yeast chromosomal element and viruses (baculovirus, papovavirus, vaccinia virus, adenovirus, avian poxvirus, pseudorabies virus, herpesvirus, lentivirus, retrovirus, and the like), and vectors (cosmid, phagemid, and the like) derived from combinations of those.

Preferably, the vector of an aspect of the present invention further includes sites for transcription initiation and transcription termination, and a ribosome-binding site in a transcriptional region. A coding part of a mature transcript in the vector contains (i) a transcription initiation codon AUG at the beginning of the polypeptide to be translated and (ii) a termination codon appropriately positioned at the end of that polypeptide.

The vector of an aspect of the present invention can include a promoter sequence to express the antibody of an aspect of the present invention in a host. The promoter sequence can be appropriately selected in accordance with a type of host cell.

The vector of an aspect of the present invention can include a sequence for enhancing transcription from DNA. In one embodiment, the sequence for enhancing transcription from DNA is an enhancer sequence. Examples of the enhancer include an SV40 enhancer, an early promoter enhancer of cytomegalovirus, a polyoma enhancer, an adenovirus enhancer, and the like.

The vector of an aspect of the present invention can include a sequence for stabilizing transcribed RNA. In one embodiment, the sequence for stabilizing transcribed RNA can be a polyA addition sequence (polyadenylation sequence, polyA). Examples of the polyA addition sequence include a polyA addition sequence derived from a growth hormone gene, a polyA addition sequence derived from a bovine growth hormone gene, a polyA addition sequence derived from a human growth hormone gene, a polyA addition sequence derived from an SV40 virus, a polyA addition sequence derived from a human or rabbit β-globin gene, and the like.

In a case where the polynucleotide of an aspect of the present invention is used as a treatment agent, it is preferable that the polynucleotide is inserted into a predetermined expression vector. In the case where the polynucleotide of an aspect of the present invention is thus inserted into a predetermined expression vector, for example, it is possible to carry out delivery of the polynucleotide to a target site more efficiently and it is also possible to increase uptake efficiency of the polynucleotide into a cell in the target site. Consequently, it is possible to effectively treat a disease which is subject to the present invention.

Such a vector has been developed in the field of drug delivery system (DDS), and a vector having intended delivery and expression effects can be selected as appropriate. Examples of such a vector include, but not limited to, viral vectors (e.g., retroviral vector, adenoviral vector, adeno-associated viral vector, and the like) and DNA vectors (e.g., pUMVC4a, and the like).

The vector of an aspect of the present invention described above can be prepared by a known method. Examples of such a method include a method described in an implementation manual attached to a kit for preparing a vector, methods described in various guidance documents, and the like.

### [4. Treatment agent for fibrotic disease]

The treatment agent for a fibrotic disease in accordance with an embodiment of the present invention (hereinafter referred to as "treatment agent of an aspect of the present invention") contains, as an active ingredient, the anti-integrin α11 monoclonal antibody of 1. above, the polynucleotide of 2. above or the vector of 3. above (hereinafter, sometimes referred to as "antibody, etc. of an aspect of the present invention").

The treatment agent of an aspect of the present invention includes the antibody of an aspect of the present invention, the polynucleotide of an aspect of the present invention, or the vector of an aspect of the present invention. Therefore, a fibrotic disease can be effectively treated by an action of the antibody, polynucleotide, or vector.

In this specification, "a fibrotic disease" means a disease caused due to tissue fibrosis itself and a tissue fibrosis condition associated with other disease or condition. Examples of the fibrotic disease include pulmonary fibrosis, hepatic fibrosis, nephrosclerosis, cardiomyopathy, cirrhosis, pancreatic fibrosis, scleroderma, and the like. The present invention also covers fibrosis (e.g., tissue fibrosis in a tumor) caused in a tissues in association with any disease or condition.

In this specification, "treatment" means giving an act that produces a treatment effect with respect to a subject in need of treatment. The treatment effect encompasses a prophylactic effect and a therapeutic effect and can be, for example, effects shown below.
(1) Prevent a development of or reduce a risk of one or more symptoms of a disease as compared with a case where no drug is administered.
(2) Prevent a relapse of or reduce a risk of one or more symptoms of a disease as compared with a case where no drug is administered.
(3) Prevent a sign of or reduce a risk of one or more symptoms of a disease as compared with a case where no drug is administered.
(4) Reduce a severity of one or more symptoms of a disease as compared with a case where no drug is administered.
(5) Prevent an increase in severity of or progression of one or more symptoms of a disease as compared with a case where no drug is administered.
(6) Reduce an increase speed in severity of or a progression speed of one or more symptoms of a disease as compared with a case where no drug is administered.

Thus, the treatment agent of an aspect of the present invention encompasses both a prophylactic agent and a therapeutic agent.

The treatment agent of an aspect of the present invention can contain, as active ingredients, other active ingredients other than the antibody, etc. of an aspect of the present invention. Such other active ingredients are preferably those which can be combined with the antibody, etc. of an aspect of the present invention to bring about a higher treatment effect, as compared with a case where the antibody, etc. of an aspect of the present invention is used alone. Other active ingredients contained in the treatment agent of an aspect of the present invention can be one kind or two or more kinds.

The treatment agent of an aspect of the present invention can include pharmaceutically acceptable carrier, diluent, excipient, and the like. The pharmaceutically acceptable carrier, diluent, excipient, and the like are well known in the pharmaceutical field and are described in, for example, Remington's Pharmaceutical Sciences, Mack Publishing Co. (A.R. Gennaro, ed., 1985). The pharmaceutically acceptable carrier, diluent, excipient can be easily selected by those skilled in the art in accordance with a route of administration of a drug and a standard pharmaceutical practice. The treatment agent of an aspect of the present invention can further include a binder, a lubricant, a suspending agent, a coating agent, a solubilizer, and/or the like.

The treatment agent of an aspect of the present invention is administered through any route of administration which is effective in the treatment. The treatment agent of an aspect of the present invention can be administered, for example, orally, intramuscularly, intravenously, subcutaneously, intraabdominally or percutaneously to a subject to be treated. An administration form can be, for example, an injectable formulation, a capsule, a tablet, granules, or the like.

The subject of treatment with the treatment agent of an aspect of the present invention is not particularly limited, provided that the subject is in need of treatment. Examples of the subject to be treated with the present treatment agent include mammals, e.g., a human, a mouse, a guinea pig, a hamster, a rat, a mouse, a rabbit, a pig, a sheep, a goat, a cattle, a horse, a cat, a dog, a marmoset, a monkey, a chimpanzee, and the like.

A method of administering the treatment agent of an aspect of the present invention is not particularly limited and can be any method used in this field. Formulation of the treatment agent of an aspect of the present invention can be appropriately set by those skilled in the art in accordance with severity of a disease, an active ingredient, a route of administration, and the like.

### [5. Use for the manufacture of treatment agent for fibrotic disease]

An embodiment of the present invention provides use of the anti-integrin α11 monoclonal antibody of 1. above, the polynucleotide of 2. above or the vector of 3. above for the manufacture of the treatment agent for a fibrotic disease.

The antibody, etc. of an aspect of the present invention has a series of activity associated with inhibition of binding between an integrin α11 and collagen as described above. Therefore, the antibody, etc. of an aspect of the present invention is applicable for the manufacture of the treatment agent for a fibrotic disease.

### [6. Diagnostic agent]

The diagnostic agent for a fibrotic disease in accordance with an embodiment of the present invention (hereinafter referred to as "diagnostic agent of an aspect of the present invention") contains the anti-integrin α11 monoclonal antibody of 1. above, the polynucleotide of 2. above or the vector of 3. above.

The diagnostic agent of an aspect of the present invention contains the antibody, etc. of an aspect of the present invention which binds to an integrin α11, and therefore the diagnostic agent can be applied to diagnose a disease (in particular, a fibrotic disease) related to an integrin α11.

The "fibrotic disease" is as described in 4. above.

A method of using the diagnostic agent of an aspect of the present invention is not particularly limited. A diagnosis of the disease can be carried out by, for example, checking and comparing (i) a binding aspect of a cell, blood, serum, a body fluid, a pathological section, or the like in a test subject with the antibody of an aspect of the present invention, and (ii) a binding aspect of a cell, blood, serum, a body fluid, a pathological section, or the like in a normal subject with the antibody of an aspect of the present invention.

A detection method employed in the diagnostic agent of an aspect of the present invention is not particularly limited. For example, radioimmunoassay, enzymatic immunoassay, fluorescent immunoassay, luminescent immunoassay, immunoprecipitation, immunonephelometry, or the like is used. These methods can be carried out by any method in this field.

In the diagnostic agent of an aspect of the present invention, it is possible to add a marker to the antibody, etc. of an aspect of the present invention. The marker is not particularly limited and, for example, a fluorochrome, an enzyme, a coenzyme, a chemiluminescent substance, a radioactive substance, or the like is used. Specifically, a radio isotope (³²P, ¹⁴C, ¹²⁵I, ³H, ¹³¹I, or the like), fluorescein, rhodamine, dansyl chloride, umbelliferone, luciferase, peroxidase, alkaline phosphatase, β-galactosidase, β-glucosidase, horseradish peroxidase, glucoamylase, lysozyme, saccharide oxidase, microperoxidase, biotin, and the like can be used. These markers can be appropriately selected depending on a purpose of diagnosis and the like.

Note that the diagnostic agent of an aspect of the present invention also contains a reagent for PET, a reagent or material used in experimentation.

### [7. Other]

Another embodiment of the present invention provides a method of treating a fibrotic disease, the method including administering, to a subject in need of treatment, an anti-integrin α11 monoclonal antibody of 1. above, a polynucleotide of 2. above or a vector of 3. above.

The "subject in need of treatment" is not particularly limited and can be a patient suffering from a fibrotic disease or a potential patient at risk of suffering from the fibrotic disease.

In a case where the polynucleotide of 2. above or the vector of 3. above is administered, the anti-integrin α11 monoclonal antibody is expressed in the body of the subject to which the polynucleotide or vector has been administered, so that the fibrotic disease can be treated.

The "fibrotic disease" is as described in 4. above.

Another embodiment of the present invention provides the anti-integrin α11 monoclonal antibody of 1. above, the polynucleotide of 2. above or the vector of 3. above for use in treatment of a fibrotic disease.

The present invention is not limited to the embodiments, but can be altered by a skilled person in the art within the scope of the claims. The present invention also encompasses, in its technical scope, any embodiment derived by combining technical means disclosed in differing embodiments.

### Examples

The following description will discuss the present invention in further detail with reference to Examples. Note, however, that the present invention is not limited to those Examples.

### (1. Anti-integrin α11 monoclonal antibody)

### [Immunization of chicken]

A gene which encodes a mouse integrin α11 I-domain (collagen binding domain, hereinafter referred to as "mouse I-domain") and a gene which encodes a chicken antibody L-chain leader sequence were linked to each other by overlapped PCR and were then cloned into a pcDNA3.1 myc-His A vector which is an expression vector for a mammal cell. The vector was then introduced into a human embryonic kidney cell line 293F cell to express and secrete a recombinant mouse I-domain protein (His-tagged to the C-terminal). The recombinant mouse I-domain protein thus secreted was purified from a 293F cell culture supernatant using Ni-NTA agarose. After that, a GELBE adjuvant and manganese chloride (final concentration of 1 mM) were mixed, and immunization of a chicken was carried out four times in total.

Sequences of various primers used in this Example are summarized in Table 1 below.

### [Table 1]

**(Table 1)**

| (1. Anti-integrin α11 monoclonal antibody) | | | |
|---|---|---|---|
| For amplifying mouse integrin α11 I-domain | moa11-F | CCTGGTGCAGGCACAG ACTTACATGGACATC | SEQ ID NO: 9 |
| | moa11-I-R-Agel | GCACCGGTCATCTCCA GCCCAAAAG | SEQ ID NO: 10 |
| For amplifying chicken antibody L-chain leader sequence | VL-L-F-BamHI | ATGGATCCGCCATGGC CTGGGCTCCTCTCCT | SEQ ID NO: 11 |
| | VL-L-R | TGCCTGCACCAGGGAA CCTG | SEQ ID NO: 12 |
| For preparing scFv phage antibody library (for VH amplification) | CHB | CTGATGGCGGCCGTGA CGTT | SEQ ID NO: 13 |
| | CHSF | TCCACCTGTCGACACG GTGACTTCGGT | SEQ ID NO: 14 |
| For preparing scFv phage antibody library (for VL amplification) | CLSB | TCTGACGTCGCGCTGA CTCAGCC | SEQ ID NO: 15 |
| | CLF | ATTAGCGCGCTTAAGGA CGGTCAGGGTT | SEQ ID NO: 16 |
| For preparing scFv phage antibody library (for linker) | Linker | CCGTGTCGACAGGTGG AGGCGGCTCTGGTGGC GGTGGCAGTGGCGGCG GAGGTTCTGACGTCGC GCTGA | SEQ ID NO: 17 |
| | Linker-comp | TCAGCGCGACGTCAGA ACCTCCGCCGCCACTG CCACCGCCACCAGAGC CGCCTCCACCTGTCGA CACGG | SEQ ID NO: 18 |
| For preparing scFv phage antibody library (for scFv amplification) | CRB | TATCTGATGGCGGCCG TGA | SEQ ID NO: 19 |
| | CRF | GACTATATTAGCGCGCT TAAGG | SEQ ID NO: 20 |
| Primer for determining scFv antibody base sequence | M13R | GGAAACAGCTATGACCA TG | SEQ ID NO: 21 |
| | SLP1 | AAGAAGCACACGACTG A | SEQ ID NO: 22 |
| For introducing human α11 | V144I-F | ATTGTCCTGGATGGCTC CAACAG | SEQ ID NO: 23 |
| | V144I-R | AATGATGTCCATGTAGG | SEQ ID NO: |
| mutation | | TCTG | 24 |
| | E205A-F | GCAGCTGCCAGCCACA TTGAGCAG | SEQ ID NO: 25 |
| | E205A-R | CACCACATCTTTTACAG ACCTG | SEQ ID NO: 26 |
| | F222Y-F | TACGGCATTGAATTTGC ACGCTC | SEQ ID NO: 27 |
| | F222Y-R | TGCCGTCCGGGTCTCT GTTCCTC | SEQ ID NO: 28 |
| | Q264D-F | GATAGCGAAAGAGACA ACGTAAC | SEQ ID NO: 29 |
| | Q264D-R | CTGGATCACCTTCTCCA GGTCTG | SEQ ID NO: 30 |
| | D325E-F | GAGAGAATCTTCAGCCT GGAAGGC | SEQ ID NO: 31 |
| | D325E-R | CCCCAGGGCATCGACA ATGTCC | SEQ ID NO: 32 |
| | T338I-F | ATCTCCTTTGGGCTGGA GATG | SEQ ID NO: 33 |
| | T338I-R | CTCGTTCTTGTTGGTGC CTTCC | SEQ ID NO: 34 |

**[Table 2] (continued from Table 1)**

| (2. Binding with integrin α11 and specificity) | | | |
|---|---|---|---|
| For amplifying chicken VH | leader-VH-F | CCCCACAGGGCTGATGGCGG CCGTGACGTTGGACG | SEQ ID NO: 35 |
| | chVH-R | GGAGGAGACGATGACTTCGG T | SEQ ID NO: 36 |
| For amplifying chicken VL + mouse CK | leader-VL-F | AGGTTCCCTGGTGCAGGCAG CAGTGACTCAGCCGC | SEQ ID NO: 37 |
| | moCK-R-XbaI | ATCTAGATTAACACTCATTCC TGTTGAAGCTC | SEQ ID NO: 38 |
| For amplifying chicken antibody H-chain leader sequence | VH-L-F-HindIII | TTAAGCTTACCATGAGCCCAC TCGTCTCC | SEQ ID NO: 39 |
| | VH-L-R | CGCCATCAGCCCTGTGGGGA | SEQ ID NO: 40 |
| For amplifying chicken antibody L-chain leader | VL-L-F-HindIII | GCAAGCTTGCCATGGCCTGG GCTCCTCTC | SEQ ID NO: 41 |
| | VL-L-R | TGCCTGCACCAGGGAACCTG | SEQ ID NO: 42 |
| For amplifying mouse IgG1 CH1 | chVH-moCH1-F | GTCATCGTCTCCTCCGCCAAA ACGACACCCCC | SEQ ID NO: 43 |
| | moCH1-R | TCTTGTCCACCTTGGTGCTGC | SEQ ID NO: 44 |

| (6. Tissue fibrosis inhibitory activity (in vitro)) | | | |
|---|---|---|---|
| (Experiment 1) | | | |
| For amplifying rat α-SMA gene (Acta2) | RatActa 2-qF | GACAATGGCTCCGGGCTCTG T | SEQ ID NO: 45 |
| | RatActa 2-qR | TGCCGTGTTCTATCGGATAC | SEQ ID NO: 46 |
| For amplifying rat Rps18 gene (control gene) | RatRps1 8-qF | TGTGGTGTTGAGGAAAGCAG | SEQ ID NO: 47 |
| | RatRps1 8-qR | TGGCCAGAACCTGGCTATAC | SEQ ID NO: 48 |

| (Experiment 2) | | | |
|---|---|---|---|
| For amplifying human α-SMA gene (Acta2) | HuActa2 -qF | TTCAATGTCCCAGCCATGTA | SEQ ID NO: 49 |
| | HuActa2 -qR | GAAGGAATAGCCACGCTCAG | SEQ ID NO: 50 |
| For amplifying human Rps18 gene (control gene) | HuRps1 8-qF | GAGGATGAGGTGGAACGTGT | SEQ ID NO: 51 |
| | HuRps1 8-qR | TCTTCAGTCGCTCCAGGTCT | SEQ ID NO: 52 |

| (Experiment 3) | | | |
|---|---|---|---|
| For amplifying mouse α-SMA gene (Acta2) | MoActa2 -qF | AATGGCTCTGGGCTCTGTAA | SEQ ID NO: 53 |
| | MoActa2 -qR | CTCTTGCTCTGGGCTTCATC | SEQ ID NO: 54 |
| For amplifying mouse GAPDH gene (control gene) | MoGAPD H-qF | TGTGTCCGTCGTGGATCTGA | SEQ ID NO: 55 |
| | MoGAPD H-qR | TTGCTGTTGAAGTCGCAGGA G | SEQ ID NO: 56 |

### [Preparation of antibody library]

A spleen was taken out from the chicken after the final immunization, and lymphocytes were isolated. After that, RNA was extracted to synthesize cDNA, and an scFv phage antibody library was prepared. Preparation of the phage antibody library was carried out in accordance with the procedure disclosed in Nakamura N et al., J Vet Med Sci. 2004 Jul; 66(7): 807-14.

### [Antibody screening]

The antibody was screened by a cell panning method to obtain an antibody responsive to an integrin α11 expressed on a cell membrane. Specifically, a phage antibody library and a hamster-ovary cell line (CHO cell: cell line that does not have an endogenous integrin α11) were mixed to remove the phage antibody adsorbed on the CHO cell. Subsequently, the remaining phage antibody was caused to react with a mutant human integrin α11 introduced CHO cell. After washing the cell with an organic solvent, the bound phage was collected and infected with E. coli. After four times of panning, reactivity of the library with respect to the CHO cell and the human integrin α11 introduced CHO cell was confirmed by FACS. Reaction conditions for FACS using the phage antibody libraries (and a phage antibody clone described later) are as follows: That is, 50 µl of the phage antibody solution as a primary antibody was added to the cell to react with the cell on ice for 25 minutes. After washing, FITC labeled anti-mouse Igκ antibody as a secondary antibody was added to the cell at a concentration of 5 µg/ml to react with the cell on ice for 15 minutes. Cell panning was carried out in accordance with the procedure disclosed in Giordano RJ et al., Nat Med. 2001 Nov; 7(11): 1249-53.

The mutant human integrin α11 is a mutant of human α11 in which, by PCR mutagenesis, valine at position of 144 was substituted with isoleucine, glutamic acid at position of 205 was substituted with alanine, phenylalanine at position of 222 was substituted with tyrosine, glutamine at position of 264 was substituted with aspartic acid, aspartic acid at position of 325 was substituted with glutamic acid, and threonine at position of 338 was substituted with isoleucine. Before the mutagenesis, an amino acid sequence of a human integrin α11 matches an amino acid sequence of the corresponding mouse α11 and, after the mutagenesis, the amino acid sequence of a human integrin α11 has been replaced with an amino acid sequence of the corresponding chicken α11.

### [Monocloning and antibody sequencing]

From the third panning library, 48 phage antibody clones were expressed, and reactivity to the CHO cell and the human integrin α11 introduced CHO cell was analyzed by FACS. Base sequences of 18 clones out of 47 clones reacted only with the human integrin α11 introduced CHO cell were determined. Consequently, the antibody was found to converge to one clone.

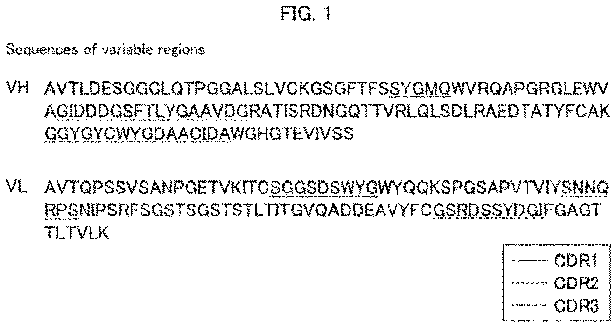

Amino acid sequences of a heavy chain variable region and a light chain variable region (including sequences of respective CDRs) of the antibody fragment (scFv) isolated above were also determined (Fig. 1, Table 2).

### [Table 3]

**(Table 2)**

| Site of antibody | Amino acid sequence | SEQ ID Number |
|---|---|---|
| Heavy chain variable region CDR1 | SYGMQ | SEQ ID NO: 1 |
| Heavy chain variable region CDR2 | GIDDDGSFTLYGAAVDG | SEQ ID NO: 2 |
| Heavy chain variable region CDR3 | GGYGYCWYGDAACIDA | SEQ ID NO: 3 |
| Light chain variable region CDR1 | SGGSDSWYG | SEQ ID NO: 4 |
| Light chain variable region CDR2 | SNNQRPS | SEQ ID NO: 5 |

| Light chain variable region CDR3 | GSRDSSYDGI | SEQ ID NO: 6 |
|---|---|---|
| Heavy chain variable region | AVTLDESGGGLQTPGGALSLVCKGSGFT FSSYGMQWVRQAPGRGLEWVAGIDDDG SFTLYGAAVDGRATISRDNGQTTVRLQLS DLRAEDTATYFCAKGGYGYCWYGDAACI DAWGHGTEVIVSS | SEQ ID NO: 7 |
| Light chain variable region | AVTQPSSVSANPGETVKITCSGGSDSWY GWYQQKSPGSAPVTVIYSNNQRPSNIPSR FSGSTSGSTSTLTITGVQADDEAVYFCGS RDSSYDGIFGAGTTLTVLK | SEQ ID NO: 8 |

### (2. Binding with integrin α11 and specificity)

### [IgG conversion of antibody]

A chicken antibody VH and a VL gene were separately PCR-amplified using the scFv antibody gene of 1. above as a template. Subsequently, chicken-mouse chimeric IgG 1 expression vectors were respectively prepared (i) by linking a chicken antibody H-chain leader sequence of VH with a mouse IgG1 constant region by overlapping PCR and (ii) by linking a chicken antibody L-chain leader sequence of VL with a mouse Igκ region by overlapping PCR. The H-chain expression vector and the L-chain expression vector thus prepared were introduced into a 293F cell so as to express and secrete chimeric IgG1 (His-tagged to the C-terminal, hereinafter referred to as "IgG"). The secreted IgG was purified from a 293F cell culture supernatant using Ni-NTA agarose. Recombination into the chimeric IgG antibody was carried out in accordance with the procedure disclosed in Tateishi Y et al., J Vet Med Sci 2008 Apr; 70(4): 397-400.

### [Analysis of reactivity (specificity) with respect to α11]

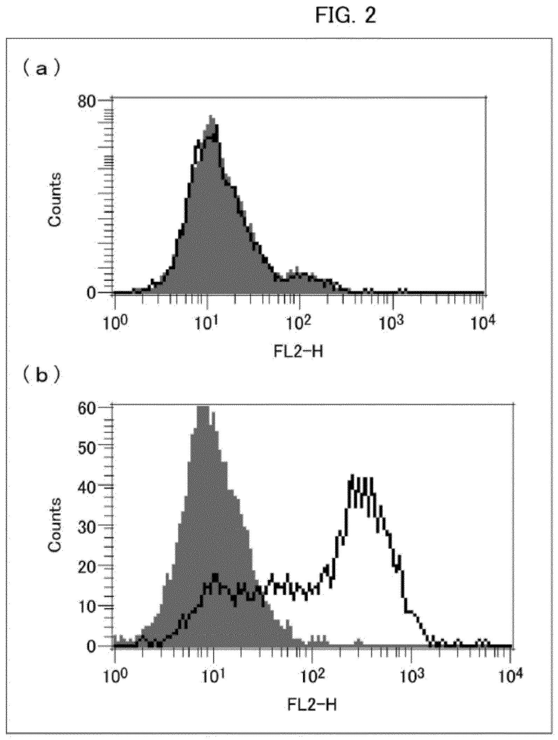

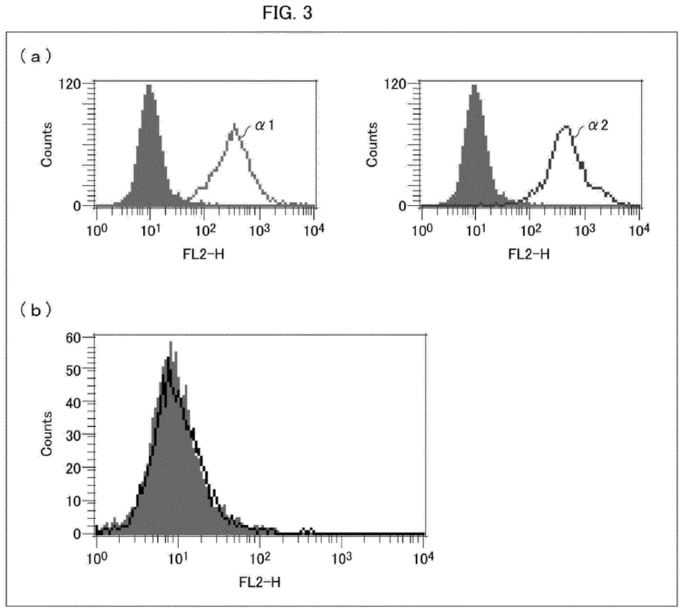

Reactivity (specificity) with respect to α11 in the antibody which has been converted into IgG was analyzed based on presence or absence of reactivity with respect to other collagen binding integrins α1, α2, and α10. First, reactivity of the antibody with respect to the CHO cell and the human integrin α11 introduced CHO cell was analyzed by FACS. Subsequently, reactivity of the antibody with respect to a human colorectal carcinoma cell line (SW480 cell: cell line expressing endogenous α1 and α2) and a human integrin α10 introduced CHO cell was analyzed by FACS. The reaction conditions of FACS using the IgG antibody are as follows. That is, the antibody as a primary antibody was added to a cell at a concentration of 5 µg/ml to react with the cell on ice for 25 minutes. After washing, PE labeled anti-mouse IgG antibody as a secondary antibody was added to the cell at a concentration of 2 µg/ml to react with the cell on ice for 15 minutes. The subsequent FACS processes were carried out under the same conditions. The results are shown in Figs. 2 through 4.

As a result, the antibody showed a reaction to an integrin α11 but did not show reactions to integrins α1, α2, and α10. Thus, the antibody was found to bind specifically to an integrin α11.

### (3. Cross-reactivity with respect to heterologous animal)

### [Reactivity with respect to rat integrin α11]

Reactivity of the antibody with respect to a rat integrin α11 introduced CHO cell was analyzed by FACS. The result is shown in Fig. 6.

### [Reactivity with respect to rat hepatic stellate cell]

It is known that a rat hepatic stellate cell is activated (myofibroblast differentiation) over the course of culturing days and that α11 expression increases, accordingly. Under the circumstances, a hepatic stellate cell was isolated from a liver of a 12-week-old Wister rat (male) and cultured. On day 5 and day 10 of culture, reactivity of the antibody with respect to the hepatic stellate cell (expression of α11) was analyzed by FACS. The rat hepatic stellate cell was isolated in accordance with the method described in Kristensen DB et al., Hepatology. 2000 Aug; 32(2): 268-77. The result is shown in Fig. 5.

Thus, the antibody showed a reaction with respect to the rat-derived integrin α11. In combination with the result of 2. above, it has been clarified that the antibody exhibits cross-reactivity with respect to a plurality of heterologous animals.

### (4. Inhibitory (neutralization) activity)

### [Cell adhesion inhibition test 1]

Rat-tail-derived type I collagen and bovine-skin-derived type III collagen were converted into solid phase in 96-well plates, respectively, overnight at a concentration of 0.4 µg/ml. 5 × 10⁴ human integrin α11 introduced mouse myoblast cell lines (C2C12 cells: cell lines that do not endogenously express all collagen binding integrins) caused to react with various concentrations (final concentrations of 0.078 to 20 µg/ml) of the antibody for 15 minutes at a room temperature were added to the wells and cultured for 1 hour, so that those cell lines adhered to each of those collagens. Adhered cells were solubilized after being fixed and stained with a 20% methanol solution containing 0.5% of crystal violet. Subsequently, absorbance was measured at 570 nm and quantified. The result is shown in Fig. 7.

The number of cells binding to collagen was reduced by adding the antibody. From this, it has been clarified that the antibody has cell adhesion inhibitory activity.

### [Cell adhesion inhibition test 2]

### <Preparation of modified antibody>

In order to prepare the modified antibody, a hot spot in a heavy chain variable region CDR3 was searched in the antibody obtained in 2. above (also referred to as "wild-type #33"). Note that the hot spot is a site that is frequently mutated during affinity maturation with respect to the antigen. As a result, amino acids at six sites were found to be the hot spots. Under the circumstances, primers were designed so that mutations were randomly introduced at those sites. The designed primer sequences are shown in Table 3.

### [Table 4]

**(Table 3)**

| Primer used in iPCR | | |
|---|---|---|
| #33VH-CD R3MUT-F | TACTTCTGCGCCAAGGGTNNSNN SNNSNNSTGTNNSNNSGGTGATG CTGCTTGTATTGACG | SEQ ID NO: 63 |
| #33VH-CD R3MUT-R | GGTGGCGGTGTCCTCAGCCCTGA GGTC | SEQ ID NO: 64 |

In Table 3, N is any one of A, T, G, and C, and S is C or G.

With use of the above designed primers, the mutant (modified) #33 DNA was amplified by inverse PCR (iPCR) using the wild-type #33 scFv phagemid vector as a template.

The template was then digested by a Dpn I treatment, and the mutant #33 DNA was circularized by self-ligation. The mutant #33 DNA thus circularized was used to prepare a heavy chain variable region CDR3 mutant #33 scFv phage antibody library, and a specific antibody was concentrated by the cell panning method described above. From the fourth panning library, 143 phage antibody clones were expressed, and base sequences and amino acid sequences of 142 clones which exhibited reactivity with respect to the human integrin α11 introduced CHO cell were determined. As a result, six clones different from the wild-type #33 were confirmed. Amino acid sequences of mutations in the six clones are shown in Fig. 13. Moreover, amino acid sequences of heavy chain variable regions CDR3 of the six clones are shown in Table 4.

### [Table 5]

**(Table 4)**

| Amino acid sequence of heavy chain variable region CDR3 of modified antibody | | |
|---|---|---|
| #33-45 | GSAGWCWMGDAACIDA | SEQ ID NO: 57 |
| #33-46 | GHTDWCWMGDAACIDA | SEQ ID NO: 58 |
| #33-52 | GEFGWCWMGDAACIDA | SEQ ID NO: 59 |
| #33-72 | GSSVDCWMGDAACIDA | SEQ ID NO: 60 |
| #33-92 | GSMGWCWMGDAACIDA | SEQ ID NO: 61 |
| #33-104 | GSEAWCWMGDAACIDA | SEQ ID NO: 62 |

### <Cell adhesion inhibition test of modified antibody>

First, wild-type #33 IgG was analyzed in terms of a cell adhesion inhibition effect under a highly-concentrated collagen solid phase condition.

Specifically, rat-tail-derived type I collagen was converted into solid phase in a 96-well plate overnight at a high concentration of 10 µg/ml. 5 × 10⁴ human integrin α11 introduced C2C12 cells caused to react with 20 µg/ml of the wild-type #33 IgG for 15 minutes at a room temperature were added to the wells and cultured for 1 hour, so that those cells adhered to the collagen. Adhered cells were solubilized after being fixed and stained with a 20% methanol solution containing 0.5% of crystal violet. Subsequently, absorbance was measured at 570 nm and quantified. The result is shown in Fig. 14. As shown in Fig. 14, under the highly-concentrated collagen condition, the wild-type #33 IgG hardly inhibited cell adhesion to collagen.

The six clones obtained above were then converted into IgG with the method described in 2. above and used in a cell adhesion inhibition test on human integrin α11 introduced C2C12 cells.

Specifically, 5 × 10⁴ human integrin α11 introduced C2C12 cells caused to react with 20 µg/ml of each modified #33 IgG (#33-45, #33-46, #33-52, #33-72, #33-92, #33-104) for 15 minutes at a room temperature were added to the wells and cultured for 1 hour, so that those cells adhered to the collagen. Adhered cells were solubilized after being fixed and stained with a 20% methanol solution containing 0.5% of crystal violet. Subsequently, absorbance was measured at 570 nm and quantified. The result is shown in Fig. 15. As shown in Fig. 15, five clones (#33-45, #33-46, #33-52, #33-92, and #33-104) inhibited cell adhesion to the collagen even under the highly-concentrated collagen condition and were found to have enhanced cellular inhibitory activity as compared with the wild-type #33 IgG.

As such, the number of cells binding to collagen was reduced by adding the modified antibody. From this, it has been clarified that the modified antibody has cell adhesion inhibitory activity.

### (5. Cell detachment activity and cell spreading inhibitory activity)

### [Cell detachment test]

Rat-tail-derived type I collagen and bovine-skin-derived type III collagen were converted into solid phase in 96-well plates, respectively, overnight at a concentration of 0.4 µg/ml. 5 × 10⁴ human integrin α11 introduced C2C12 cells were added to the wells and cultured for 45 minutes, so that those cells adhered to the collagen. Subsequently, various concentrations (final concentrations of 0.078 to 20 µg/ml) of the antibody were added and cultured for additional 1.5 hours. As in 4. above, the cells were fixed and stained and solubilized. After that, absorbance was measured at 570 nm to assess cell detachment and cell spreading. The results are shown in Figs. 8 and 9.

The number of cells binding to collagen was reduced by adding the antibody. From this, it has been clarified that the antibody has cell detachment activity. Moreover, spreading of cell was inhibited by adding the antibody. From this, it has been clarified that the antibody has cell spreading inhibitory activity involved in cell adhesion inhibition.

### (6. Tissue fibrosis inhibitory activity (in vitro))

### [Myofibroblastization inhibition test]

Myofibroblastization inhibitory activity of the antibody was evaluated while using, as an indicator, expression of an α-SMA gene which is a marker of myofibroblast.
(Experiment 1) In a manner similar to that in 3. above, a hepatic stellate cell was isolated from a rat liver and cultured. On day 3 of culture, the antibody was added at a final concentration of 20 µg/ml. The cells were collected on days 5, 7, 11 and 14 of culture, and RNA was extracted. cDNA was synthesized from the extracted RNA, and expression of the α-SMA gene (Acta2) was analyzed by quantitative PCR. The result is shown in Fig. 10.
(Experiment 2) A human breast cancer cell line Hs578T (confirmed to express α11 on a cell surface) was cultured overnight in a serum-free medium, and then the antibody was added at a final concentration of 20 µg/ml and cultured for 2 hours. Subsequently, recombinant human TGF-β1 was added at a final concentration of 5 ng/ml and cultured under a serum-free condition for additional 2 days. RNA was extracted from the cultured cells. cDNA was synthesized from the extracted RNA, and expression of the α-SMA gene (Acta2) was analyzed by quantitative PCR. The result is shown in Fig. 11.
(Experiment 3) Human integrin α11 introduced C2C12 cells cultured overnight in a serum-free medium were detached from a dish. After that, the antibody was added at a final concentration of 25 µg/ml to react with the cells at a room temperature for 15 minutes. After the reaction, 2 × 10⁵ cells were added to wells in which rat-tail-derived type I collagen was converted into solid phase overnight at a concentration of 2 µg/ml, and cultured under a serum-free condition for 24 hours. The cultured cells were collected, and RNA was extracted. cDNA was synthesized from the extracted RNA, and expression of the α-SMA gene (Acta2) was analyzed by quantitative PCR. The result is shown in Fig. 12.

From the Experiments 1 through 3 above, it has been clarified that the antibody has tissue fibrosis inhibitory activity.

### Industrial Applicability

The present invention can inhibit binding between an integrin α11 and collagen, and therefore the present invention can be widely used in fields such as medicines, agriculture, forestry and fisheries, life sciences, biotechnology, and gene therapies.

## Claims

1. An anti-integrin α11 monoclonal antibody which inhibits binding between an integrin α11 and collagen.

2. The anti-integrin α11 monoclonal antibody as set forth in claim 1 which has at least one activity selected from the group consisting of cell adhesion inhibitory activity and cell detachment activity.

3. The anti-integrin α11 monoclonal antibody as set forth in claim 1 or 2 which specifically binds to an integrin α11.

4. The anti-integrin α11 monoclonal antibody as set forth in any one of claims 1 through 3 which specifically binds to integrins α11 derived from two or more different species of mammals.

5. The anti-integrin α11 monoclonal antibody as set forth in any one of claims 1 through 4, comprising:
(a) a heavy chain variable region CDR1 which is any of the following (a1) through (a3):
(a1) a polypeptide having an amino acid sequence of SEQ ID NO: 1;
(a2) a polypeptide having an amino acid sequence in which one or more amino acids are deleted, substituted, or added in the amino acid sequence of SEQ ID NO: 1, and having a function as the heavy chain variable region CDR1;
(a3) a polypeptide having an amino acid sequence that has homology of 80% or higher with respect to the amino acid sequence of SEQ ID NO: 1, and having a function as the heavy chain variable region CDR1;
(b) a heavy chain variable region CDR2 which is any of the following (b1) through (b3):
(b1) a polypeptide having an amino acid sequence of SEQ ID NO: 2;
(b2) a polypeptide having an amino acid sequence in which one or more amino acids are deleted, substituted, or added in the amino acid sequence of SEQ ID NO: 2, and having a function as the heavy chain variable region CDR2;
(b3) a polypeptide having an amino acid sequence that has homology of 80% or higher with respect to the amino acid sequence of SEQ ID NO: 2, and having a function as the heavy chain variable region CDR2;
(c) a heavy chain variable region CDR3 which is any of the following (c1) through (c3):
(c1) a polypeptide having an amino acid sequence of SEQ ID NO: 3;
(c2) a polypeptide having an amino acid sequence in which one or more amino acids are deleted, substituted, or added in the amino acid sequence of SEQ ID NO: 3, and having a function as the heavy chain variable region CDR3;
(c3) a polypeptide having an amino acid sequence that has homology of 80% or higher with respect to the amino acid sequence of SEQ ID NO: 3, and having a function as the heavy chain variable region CDR3;
(d) a light chain variable region CDR1 which is any of the following (d1) through (d3):
(d1) a polypeptide having an amino acid sequence of SEQ ID NO: 4;
(d2) a polypeptide having an amino acid sequence in which one or more amino acids are deleted, substituted, or added in the amino acid sequence of SEQ ID NO: 4, and having a function as the light chain variable region CDR1;
(d3) a polypeptide having an amino acid sequence that has homology of 80% or higher with respect to the amino acid sequence of SEQ ID NO: 4, and having a function as the light chain variable region CDR1;
(e) a light chain variable region CDR2 which is any of the following (e1) through (e3):
(e1) a polypeptide having an amino acid sequence of SEQ ID NO: 5;
(e2) a polypeptide having an amino acid sequence in which one or more amino acids are deleted, substituted, or added in the amino acid sequence of SEQ ID NO: 5, and having a function as the light chain variable region CDR2;
(e3) a polypeptide having an amino acid sequence that has homology of 80% or higher with respect to the amino acid sequence of SEQ ID NO: 5, and having a function as the light chain variable region CDR2; and
(f) a light chain variable region CDR3 which is any of the following (f1) through (f3):
(f1) a polypeptide having an amino acid sequence of SEQ ID NO: 6;
(f2) a polypeptide having an amino acid sequence in which one or more amino acids are deleted, substituted, or added in the amino acid sequence of SEQ ID NO: 6, and having a function as the light chain variable region CDR3;
(f3) a polypeptide having an amino acid sequence that has homology of 80% or higher with respect to the amino acid sequence of SEQ ID NO: 6, and having a function as the light chain variable region CDR3.

6. The anti-integrin α11 monoclonal antibody as set forth in any one of claims 1 through 5, comprising:
(g) a heavy chain variable region which is any of the following (g1) through (g3):
(g1) a polypeptide having an amino acid sequence of SEQ ID NO: 7;
(g2) a polypeptide having an amino acid sequence in which one or more amino acids are deleted, substituted, or added in the amino acid sequence of SEQ ID NO: 7, and having a function as the heavy chain variable region;
(g3) a polypeptide having an amino acid sequence that has homology of 80% or higher with respect to the amino acid sequence of SEQ ID NO: 7, and having a function as the heavy chain variable region; and
(h) a light chain variable region which is any of the following (h1) through (h3):
(h1) a polypeptide having an amino acid sequence of SEQ ID NO: 8;
(h2) a polypeptide having an amino acid sequence in which one or more amino acids are deleted, substituted, or added in the amino acid sequence of SEQ ID NO: 8, and having a function as the light chain variable region;
(h3) a polypeptide having an amino acid sequence that has homology of 80% or higher with respect to the amino acid sequence of SEQ ID NO: 8, and having a function as the light chain variable region.

7. The anti-integrin α11 monoclonal antibody as set forth in any one of claims 1 through 4, comprising:
(a) a heavy chain variable region CDR1 which is any of the following (a1) through (a3):
(a1) a polypeptide having an amino acid sequence of SEQ ID NO: 1;
(a2) a polypeptide having an amino acid sequence in which one or more amino acids are deleted, substituted, or added in the amino acid sequence of SEQ ID NO: 1, and having a function as the heavy chain variable region CDR1;
(a3) a polypeptide having an amino acid sequence that has homology of 80% or higher with respect to the amino acid sequence of SEQ ID NO: 1, and having a function as the heavy chain variable region CDR1;
(b) a heavy chain variable region CDR2 which is any of the following (b1) through (b3):
(b1) a polypeptide having an amino acid sequence of SEQ ID NO: 2;
(b2) a polypeptide having an amino acid sequence in which one or more amino acids are deleted, substituted, or added in the amino acid sequence of SEQ ID NO: 2, and having a function as the heavy chain variable region CDR2;
(b3) a polypeptide having an amino acid sequence that has homology of 80% or higher with respect to the amino acid sequence of SEQ ID NO: 2, and having a function as the heavy chain variable region CDR2;
(c') a heavy chain variable region CDR3 which is a polypeptide having an amino acid sequence of the following Formula (1):
G(X₁X₂X₃X₄)C(X₅X₆)GDAACIDA ... (1)
wherein:
X₁ is S, H, or E,
X₂ is A, T, F, S, M, or E,
X₃ is G, D, V or A,
X₄ is W or D,
X₅ is W,
X₆ is M;
(d) a light chain variable region CDR1 which is any of the following (d1) through (d3):
(d1) a polypeptide having an amino acid sequence of SEQ ID NO: 4;
(d2) a polypeptide having an amino acid sequence in which one or more amino acids are deleted, substituted, or added in the amino acid sequence of SEQ ID NO: 4, and having a function as the light chain variable region CDR1;
(d3) a polypeptide having an amino acid sequence that has homology of 80% or higher with respect to the amino acid sequence of SEQ ID NO: 4, and having a function as the light chain variable region CDR1;
(e) a light chain variable region CDR2 which is any of the following (e1) through (e3):
(e1) a polypeptide having an amino acid sequence of SEQ ID NO: 5;
(e2) a polypeptide having an amino acid sequence in which one or more amino acids are deleted, substituted, or added in the amino acid sequence of SEQ ID NO: 5, and having a function as the light chain variable region CDR2;
(e3) a polypeptide having an amino acid sequence that has homology of 80% or higher with respect to the amino acid sequence of SEQ ID NO: 5, and having a function as the light chain variable region CDR2; and
(f) a light chain variable region CDR3 which is any of the following (f1) through (f3):
(f1) a polypeptide having an amino acid sequence of SEQ ID NO: 6;
(f2) a polypeptide having an amino acid sequence in which one or more amino acids are deleted, substituted, or added in the amino acid sequence of SEQ ID NO: 6, and having a function as the light chain variable region CDR3;
(f3) a polypeptide having an amino acid sequence that has homology of 80% or higher with respect to the amino acid sequence of SEQ ID NO: 6, and having a function as the light chain variable region CDR3.

8. A polynucleotide comprising a base sequence which encodes an anti-integrin α11 monoclonal antibody recited in any one of claims 5 through 7.

9. A vector comprising a polynucleotide recited in claim 8.

10. A treatment agent for a fibrotic disease, said treatment agent comprising:
an anti-integrin α11 monoclonal antibody recited in any one of claims 1 through 7 as an active ingredient;
a polynucleotide recited in claim 8 as an active ingredient; or
a vector recited in claim 9 as an active ingredient.

11. The treatment agent as set forth in claim 10, wherein the fibrotic disease is at least one disease selected from the group consisting of pulmonary fibrosis, hepatic fibrosis, nephrosclerosis, cardiomyopathy, cirrhosis, pancreatic fibrosis, and scleroderma.

12. Use of an anti-integrin α11 monoclonal antibody recited in any one of claims 1 through 7, a polynucleotide recited in claim 8, or a vector recited in claim 9 for the manufacture of a treatment agent for a fibrotic disease.

13. A diagnostic agent for a fibrotic disease, said diagnostic agent comprising:
an anti-integrin α11 monoclonal antibody recited in any one of claims 1 through 7;
a polynucleotide recited in claim 8; or
a vector recited in claim 9.
